# FASCICULE DE BREVET EUROPEEN

(11) **EP 1 315 739 B1**
(45) Date de publication et mention de la délivrance du brevet: **18.08.2004**
(21) Numéro de dépôt: 01967454.8
(22) Date de dépôt: 07.09.2001
(51) Int. Cl.: C07K 1/107, A61K 47/48, C07C 233/03, C07D 209/32, A61K 39/395

(54) **PROCEDE DE COUPLAGE, EN SOLUTION, ENTRE UN PEPTIDE ET UN VECTEUR LIPOPHILE ET SES APPLICATIONS**
VERFAHREN ZUR KUPPLUNG IN LÖSUNG ZWISCHEN EINEM PEPTID UND EINER LIPOPHILEN VERBINDUNG, UND VERWENDUNGEN DAVON
METHOD FOR BINDING, IN SOLUTION, A PEPTIDE AND A LIPOPHILIC VECTOR AND USES THEREOF

(30) Priorité: 08.09.2000 FR 0011451
(43) Date de publication de la demande: 04.06.2003
(73) Titulaire: INSTITUT PASTEUR DE LILLE Etablissement Public, 59019 Lille Cédex (FR); CENTRE NATIONAL DE LA RECHERCHE SCIENTIFIQUE (CNRS), 75794 Paris Cedex 16 (FR)
(72) Inventeur: BONNET, Dominique, F-59000 Lille (FR); BOUREL, Line, F-59000 Lille (FR); MELNYK, Oleg, F-59112 Annoeullin (FR); GRAS-MASSE, Hélène, F-59710 Merignies (FR)
(74) Mandataire: Lepeudry-Gautherat, Thérèse
(86) Numéro de dépôt international: PCT/FR2001/002787
(87) Numéro de publication internationale: WO 2002/020558

(56) Documents cités:
- DATABASE CHEMABS [en ligne] CHEMICAL ABSTRACTS SERVICE, COLUMBUS, OHIO, US; FRUCHART, JEAN-SEBASTIEN ET AL: "A new tartaric acid-based linker for the synthesis of C-terminal peptide.alpha.-oxo-aldehydes" retrieved from STN Database accession no. 134:237782 CA XP002172766 & PEPT. NEW MILLENNIUM, PROC. AM. PEPT. SYMP., 16TH (2000), MEETING DATE 1999, 104-106. EDITOR(S): FIELDS, GREGG B.;TAM, JAMES P.; BARANY, GEORGE. PUBLISHER: KLUWER ACADEMIC PUBLISHERS, DORDRECHT, NETH., 2000,
- C GRANDJEAN ET AL.: "Convergent synthesis of D-(-)-quinic and shikimic acid-containing dendrimers as potential C-lectin ligands by sulfide ligation of unprotected fragments" JOURNAL OF THE CHEMICAL SOCIETY, PERKIN TRANSACTIONS 1., no. 10, octobre 1999 (1999-10), pages 2967-2975, XP002137985 CHEMICAL SOCIETY. LETCHWORTH., GB ISSN: 1472-7781
- O MELNYK ET AL.: "Synthesis of lipopeptides using hydrazone chemical ligation" JOURNAL OF PEPTIDE RESEARCH., vol. 52, no. 9, septembre 1998 (1998-09), pages 180-184, XP000774264 MUNKSGAARD, COPENHAGEN., DK ISSN: 0367-8377 cité dans la demande
- J SHAO & J P TAM: "Unprotected peptides as building blocks for the synthesis of peptide dendrimers with oxime, hydrazone, and thiazolidine linkage" JOURNAL OF THE AMERICAN CHEMICAL SOCIETY., vol. 117, no. 14, 12 avril 1995 (1995-04-12), pages 3893-3899, XP002172763 AMERICAN CHEMICAL SOCIETY, WASHINGTON, DC., US ISSN: 0002-7863 cité dans la demande
- D BONNET ET AL.: "A novel lipophilic glyoxylic acid derivative for the lipidation of peptides using salt-free hydrazone ligation" TETRAHEDRON LETTERS., vol. 41, décembre 2000 (2000-12), pages 10003-10007, XP002172764 ELSEVIER SCIENCE PUBLISHERS, AMSTERDAM., NL ISSN: 0040-4039
- D BONNET ET AL.: "Synthesis of an amphiphilic aldehyde using as a key step the condensation of a lipophilic glyoxylic acid derivative with tris(hydroxymehtyl)aminomethane" TETRAHEDRON LETTERS., vol. 42, no. 10, 4 mars 2001 (2001-03-04), pages 1875-1877, XP002172765 ELSEVIER SCIENCE PUBLISHERS, AMSTERDAM., NL ISSN: 0040-4039

## Description

L'invention se rapporte à un procédé de couplage, en solution, entre au moins un composé peptidique et au moins un vecteur lipophile portant une fonction aldéhyde, aux vecteurs lipophiles susceptibles d'être utilisés dans ce procédé, aux lipopeptides ainsi obtenus et aux applications de ce procédé, notamment pour l'obtention de médicaments.

Le problème de l'entrée dans les cellules vivantes de différentes substances dotées de propriétés pharmacologiques revêt une grande importance sur le plan thérapeutique. Les peptides synthétiques et les oligonucléotides traversent difficilement la membrane cellulaire. Une approche intéressante pour améliorer leur capacité à pénétrer dans la cellule est de les modifier par une partie lipophile. Ainsi, il a été montré qu'un peptide modifié par une simple chaîne aliphatique est capable de pénétrer au sein de la cellule par transfert passif au travers de la membrane et d'interagir avec sa cible intracytoplasmique. Les lipopeptides représentent donc des molécules intéressantes pour vectoriser un motif fonctionnel au sein de la cellule.

La synthèse de lipopeptides peut être réalisée, par exemple, en couplant un acide gras à un peptide en phase solide, comme décrit notamment par K. THIAM et al. dans Biochemical and Biophysical Research Communications, 1998, 253, 639-647 et dans Journal of Medicinal Chemistry, 1999, 42, 3732-3736, ainsi que par C. KLINGUER et al. dans Vaccine, 2000, 18, 259-267. En fin de synthèse, des étapes de clivage de la liaison peptide/support solide et de déprotection des chaînes latérales du peptide par un acide fort doivent être réalisées. Ce traitement limite de façon importante le choix de la partie lipophile ; il interdit notamment l'utilisation d'acides gras insaturés. En outre, la purification des lipopeptides par chromatographie liquide haute performance en phase inverse, nécessaire après le clivage de la liaison peptide/support, est difficile et conduit à des rendements faibles eu égard aux nombreuses impuretés qui sont présentes en fin de synthèse.

Il a également été proposé de coupler, en solution, une protéine à un groupement palmitoyl-coenzyme A, ce dernier étant introduit au niveau du groupement thiol d'une cystéine. Un tel couplage se traduit par la formation d'un lien thioester, qui présente l'inconvénient d'être peu stable. D'autre part, cette stratégie est limitée à la modification de certaines protéines par le palmitoyl-coenzyme A et ne peut pas être généralisée à la synthèse de lipopeptides.

Les stratégies actuelles pour la synthèse de lipopeptides consistent également à mettre en oeuvre des réactions de ligation chimique. La ligation chimique permet de lier, en solution et dans des conditions extrêmement douces, deux structures peptidiques préalablement purifiées et totalement déprotégées.

Il a ainsi été proposé de lier, dans un tampon aqueux, un acide gras à un peptide par un lien disulfure. Cependant, le lien disulfure pose de nombreux problèmes ; un tel lien est en effet peu stable et susceptible d'être dégradé en présence de thiols, d'où la nécessité d'éviter la contamination des solvants utilisés pour la solubilisation des produits par des thiols, ainsi que l'impossibilité d'introduire une cystéine dans la séquence peptidique à vectoriser. L'utilisation de la chimie des thiols nécessite, en outre, de travailler sous atmosphère inerte dans le but d'éviter l'oxydation des thiols.

W. ZENG et al. (J. Pept. Sc., 1996, 2, 66-72) ont également proposé de lier, en solution, un peptide totalement déprotégé et préalablement purifié à une structure lipidique polyfonctionnelle liée à un peptide, et ce par le biais d'un lien oxime. La partie lipophile est introduite sur une séquence peptidique en phase solide, une telle méthode présentant les inconvénients mentionnés ci-dessus, à savoir la limitation au niveau du choix de la partie lipophile et les difficultés liées à la purification de la structure lipidique.

De façon similaire, O. MELNYK et al. (J. Peptide Res., 1998, 52, 180-184) ont décrit la ligation, en solution et par un lien hydrazone, entre un peptide portant une chaîne lipophile et une fonction aldéhyde et un autre peptide modifié au niveau de la chaîne latérale de lysine par un groupement hydrazino. Le lien hydrazone est réalisé en solution, mais le composé lipophile, de nature peptidique, est synthétisé en phase solide et les limitations sont identiques à celles évoquées précédemment.

Par ailleurs, C. KLINGUER et al. (Tetrahedron Letters, 1996, 37, 40, 7259-7262) ont décrit la ligation, dans un mélange eau/acétonitrile, entre le cyclohexanecarboxaldéhyde et un peptide portant une fonction hydrazine. Le procédé décrit par ces auteurs ne permet toutefois pas d'obtenir des composés stables, et donc utilisables pour la vectorisation de principes actifs : en effet, le lien hydrazone formé entre l'hydrazine et l'aldéhyde est instable dans une large gamme de pH.

Enfin, D. BONNET et al. (Tetrahedron Letters, 2000, 41, 45-48) ont décrit la ligation, en solution et par un lien hydrazide, de peptides fonctionnalisés par un résidu α-hydrazinoacétique avec des esters activés d'acides gras. Cette méthode, décrite également dans la Demande de Brevet français n° 99 10626, permet l'introduction d'acides gras complexes sur des peptides. Cependant, une purification par HPLC est nécessaire en fin de synthèse pour séparer le lipopeptide des sels, des réactifs d'activation et des impuretés (notamment le peptide polyacylé) présents dans le milieu réactionnel. Cette purification est simplifiée par la qualité du couplage en solution, mais néanmoins nécessaire pour les raisons invoquées ci-dessus. Ce procédé de couplage n'est donc pas adapté à la synthèse d'un mélange de lipopeptides, tels que ceux utilisés dans certains vaccins (H. GAHERY-SEGARD et al., Journal of Virology, 2000, 74, n° 4, 1694-1703 ; C. KLINGUER et al., Vaccine, ibid).

Au vu des inconvénients de l'état de la technique mentionnés ci-dessus, les Inventeurs se sont donnés pour but de pourvoir à une nouvelle stratégie de synthèse de lipopeptides et, de façon générale, de peptides modifiés par différents composés de nature lipidique, par ligation chimique en solution.

Cette nouvelle stratégie de synthèse doit notamment répondre aux critères suivants :
- le couplage du composé de nature lipidique au peptide s'effectue en solution,
- le couplage s'effectue à partir d'un peptide totalement déprotégé, la réaction étant chimiosélective,
- les conditions réactionnelles du couplage permettent l'utilisation de dérivés d'acides gras, y compris de dérivés d'acides gras complexes mono- et polyinsaturés, et de dérivés du cholestérol commerciaux,
- le lien formé au cours du couplage est très stable dans une large gamme de pH,
- le couplage permet l'obtention de mélanges de lipopeptides directement utilisables sans étape de purification préalable.

Les Inventeurs se sont également donnés pour but de pourvoir à des lipopeptides, susceptibles d'être obtenus par ligation chimique, dans lesquels le lien lipide/peptide est très stable et ne présente pas les inconvénients des liens disulfures de l'Art antérieur.

Ces buts sont atteints par la création d'un lien hydrazone entre un peptide et un vecteur lipophile de nature non peptidique, lors d'une synthèse convergente en solution.

L'invention a pour objet un procédé de couplage, en solution, entre au moins un composé peptidique et au moins un vecteur lipophile portant une fonction aldéhyde, ledit couplage comprenant une étape de réalisation d'un lien hydrazone entre ledit composé peptidique et ledit vecteur lipophile, lequel procédé est caractérisé en ce que ledit vecteur lipophile est de nature non peptidique et répond à la formule générale (I) :

[(R¹)(R²)ᵢ]D-CHO (I)

dans laquelle :
- i représente 0 ou 1,
- dans le cas où i est égal à 0, D représente une liaison,
- dans le cas où i est égal à 1, D représente un hétérocycle saturé, insaturé ou aromatique, mono- ou polycyclique, et
- R¹ et R², qui peuvent être identiques ou différents, représentent chacun un groupement de formule L-f-E-f' où L représente un résidu d'un lipide, E représente un bras espaceur, et f et f' représentent des fonctions liant, respectivement, L à E et E à D.

De façon particulièrement avantageuse, le procédé selon l'invention, réalisé en solution, permet d'éviter une étape de clivage du support du composé peptidique modifié ainsi obtenu, clivage qui limite de façon importante le choix de la partie lipophile couplée au composé peptidique, comme cela a été évoqué précédemment.

Selon un mode de mise en oeuvre avantageux du procédé conforme à l'invention, ce dernier comprend les étapes suivantes :
a) préparation d'au moins un composé peptidique totalement déprotégé portant, soit au niveau de son extrémité N-terminale, soit au niveau de l'extrémité de la chaîne latérale d'une lysine ou d'une ornithine éventuellement présente à un endroit quelconque de la séquence peptidique, 1 à 4 groupements dérivés d'hydrazine de formule -CO-CHR'-NR-NH₂, où R et R' représentent, indépendamment l'un de l'autre, un atome d'hydrogène ou un groupement alkyle saturé ou insaturé, linéaire, ramifié ou cyclique, comprenant de 1 à 10 atomes de carbone, ainsi qu'éventuellement 1 à 3 hétéroatomes choisis parmi l'oxygène, le soufre et l'azote, et pouvant être substitué par 1 à 6 groupements choisis parmi les groupes hydroxy, alkoxy, aryloxy, amino, aminoalkyle, aminoaryle, thio, thioalkyle, carbonyle, carboxyle, guanidino et carboxamido ;
b) réaction, en solution, du(des)dit(s) composé(s) peptidique(s) obtenu(s) à l'étape a) avec au moins un vecteur lipophile de nature non peptidique de formule (I) telle que définie ci-dessus, porteur d'une fonction aldéhyde.

Un groupement dérivé d'hydrazine, tel que défini ci-dessus, peut être introduit, soit au niveau de l'extrémité N-terminale du peptide, soit au niveau de l'extrémité de la chaîne latérale d'une lysine ou d'une ornithine éventuellement présente à un endroit quelconque de la séquence peptidique, par tout moyen connu de l'Homme de l'Art.

Les groupements dérivés d'hydrazine portés par le composé peptidique, mentionnés lors de l'étape a) du procédé conforme à l'invention, sont de préférence des groupements α-hydrazinoacétiques, c'est-à-dire des groupements -CO-CH₂-NH-NH₂. De tels groupements peuvent, par exemple, être introduits sur le composé peptidique à l'aide de l'acide N,N'-tri(Boc)hydrazino-acétique ou de l'acide N,N'-di(Boc)hydrazinoacétique, comme décrit dans la Demande internationale PCT/FR00/02336, ainsi que dans l'article de D. BONNET et al. paru dans Tetrahedron Letters, 2000, 41, 45-48. La réaction est effectuée en phase solide, le peptide fonctionnalisé étant ensuite séparé du support solide et déprotégé selon des méthodes connues de l'Homme de l'Art.

De façon particulièrement avantageuse, la réaction de couplage entre ledit vecteur lipophile de formule (I) et ledit composé peptidique totalement déprotégé, fonctionnalisé tel que décrit ci-dessus, permet d'éviter toute étape de déprotection des chaînes latérales du composé peptidique avec un acide fort postérieurement à la réaction de couplage, ce qui permet d'utiliser, en tant que vecteur lipophile, des dérivés d'acides gras sensibles. Le procédé selon l'invention permet ainsi d'obtenir directement un composé peptidique modifié, c'est-à-dire couplé à un vecteur lipophile.

La réaction de couplage réalisée au cours du procédé selon l'invention (étape b) s'effectue dans des conditions opératoires très douces et ne nécessite pas de travailler dans des conditions inertes, comme c'est le cas pour certains des procédés de l'Art antérieur, notamment ceux qui consistent à coupler un peptide à un acide gras par un lien disulfure. Le couplage est efficace, rapide et s'effectue dans des conditions stoechiométriques. Il n'y a pas ou peu de sous-produits et de précurseurs non consommés, ce qui permet d'obtenir des lipopeptides directement utilisables sans avoir à être purifiés. Ceci est particulièrement avantageux dans la mesure où les lipopeptides sont des molécules difficiles à purifier par chromatographie liquide haute performance ou par d'autres méthodes (eu égard à des problèmes de solubilité, d'agrégation ...). Dans le procédé conforme à l'invention, les précurseurs peptidiques sont facilement purifiés avant d'être couplés aux vecteurs lipophiles. Ainsi, les lipopeptides obtenus ne nécessitent pas d'être purifiés.

En outre, le lien hydrazone formé, au cours de l'étape b) du procédé conforme à l'invention, entre la fonction aldéhyde du vecteur lipophile de formule (I) et le goupement dérivé d'hydrazine de formule -CO-CHR'-NR-NH₂ portée par le composé peptidique, est particulièrement stable, en tout état de cause bien plus stable qu'une fonction hydrazone formée entre un aldéhyde et un groupement hydrazine (-NH-NH₂), type de lien hydrazone décrit notamment par C. KLINGUER et al. (Tetrahedron Letters, 1996, 37, 40, 7259-7262). En effet, la présence de la fonction carbonyle, dans les groupements dérivés d'hydrazine portés par les composés peptidiques utilisés dans le cadre de la présente invention, modifie la réactivité de la fonction hydrazine et stabilise le lien hydrazone formé.

La réaction effectuée à l'étape b) du procédé conforme à l'invention est avantageusement réalisée dans un mélange d'eau (pour la solubilisation du composé peptidique) et d'au moins un solvant lipophile miscible à l'eau (pour la solubilisation du vecteur lipophile), de préférence le tert-butanol.

Le composé peptidique mis en oeuvre dans le procédé conforme à l'invention peut être sélectionné dans le groupe constitué par les peptides et les dérivés de peptides, tels que les glycopeptides, les pseudopeptides et les glycomimétiques dendrimériques de nature peptidique.

Au sens de la présente invention, on entend par « peptide » tout enchaînement de plusieurs acides aminés, quels que soient leur nature et leur nombre ; le terme « peptide » désigne donc aussi bien des oligopeptides (dipeptides ou tripeptides) que des polypeptides ou des protéines. On entend par « glycopeptides » des peptides associés, par liaison covalente, avec des glucides, qu'il s'agisse de monosaccharides (tels que des oses neutres) ou de polysaccharides. Par « pseudopeptides », on entend des peptides dont une ou plusieurs liaisons peptidiques (-CO-NH-) ont été remplacées par des liaisons non peptidiques (telles que les liaisons -CO-NH-NH-, -CH₂-NH- ou -CO-NH-O-). Par « glycomimétiques dendrimériques de nature peptidique », on entend des glycomimétiques dont la structure est à base d'acides aminés ou de leurs dérivés et se présente sous une forme hautement ramifiée, de type arborescente.

Les peptides et les dérivés de peptides utilisables dans le procédé de couplage conforme à l'invention peuvent avantageusement présenter une activité pharmacologique et un intérêt thérapeutique : il peut s'agir par exemple de neuropeptides, d'hormones peptidiques ou de substances susceptibles de moduler l'activité de récepteurs de cytokines. Le couplage de ces peptides ou dérivés de peptides à un vecteur lipophile, conformément au procédé selon l'invention, leur confère la capacité de pénétrer dans les cellules par transfert à travers la membrane cellulaire, et ce quelles que soient les cellules.

Selon un mode de mise en oeuvre avantageux du procédé conforme à l'invention, le composé peptidique utilisé est un glycomimétique dendrimérique de nature peptidique qui répond à la formule générale (II) ci-après :

(B²M)ₘ(XN)ₙA (II)

dans laquelle :
- B² répond à une ou plusieurs formules générales (a) ou (b) ci-après : 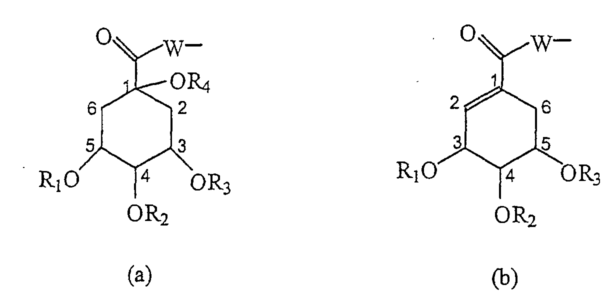
où R₁, R₂, R₃ et R₄ représentent, indépendamment les uns des autres, un atome d'hydrogène ou un groupe protecteur, et où W représente une liaison ou une chaîne carbonée linéaire, ramifiée ou cyclique, saturée ou insaturée, comprenant de 1 à 18 atomes de carbone et éventuellement de 1 à 12 atomes choisis parmi l'oxygène, le soufre et l'azote, ladite chaîne carbonée étant éventuellement substituée par 1 à 16 atomes d'halogène,
- X représente le reste d'un oligopeptide X' comprenant de 1 à 6 acides aminés,
- A représente le reste d'un composé A' au moins trifonctionnel,
- m est un nombre entier compris entre 1 et 32,
- n est un nombre entier compris entre 0 et 32, et
- M et N représentent chacun un groupe de liaison, respectivement entre B² et A quand n = 0 ou B² et X quand n est différent de 0, et entre X et A quand n est différent de 0, et comprennent, indépendamment l'un de l'autre, une fonction choisie parmi les fonctions oxime, hydrazone, amide, ester, thioester, hydrazide, hydroxamate, éther, thioéther, amine, carbonate, carbamate, thiocarbonate, thiocarbamate, urée, thiourée et thiazolidine.

Le composé de formule (II) est décrit dans la Demande internationale PCT/FR00/02194.

Au sens de la présente invention, on entend par groupe « protecteur » un groupe protecteur d'une fonction hydroxyle ou d'un diol (auquel cas deux groupes choisis parmi R₁, R₂, R₃ et R₄ peuvent être reliés de façon covalente l'un à l'autre pour former ensemble un cycle), tel que défini dans l'ouvrage Protective Groups in Organic Synthesis, T.W. GREENE et P.G.M. WUTS, Seconde Edition 1991, J. WILEY and Sons. A titre d'exemples et de façon non-limitative, on peut citer les groupes triméthylsilyle, triéthylsilyle, triisopropylsilyle, tertiobutyldiméthylsilyle, tertiobutyldiphénylsilyle, triméthylsilyléthyléther, tert-butoxyméthyle, méthoxyméthyle, benzyloxyméthyle, 2-méthoxyéthoxyméthyle, méthylthiométhyle, acétyle, ou 2',3'-diméthoxybutane-2',3'-diyle.

A titre d'exemples et de façon non-limitative, dans les formules générales (a) et (b) :
- des atomes d'halogène convenables sont le brome, le chlore et le fluor ; et
- des chaînes carbonées convenables sont des groupes alkyles (méthyle, éthyle, propyle, isopropyle, tertiobutyle, pentyle, ...), des groupes alkényles (vinyle, allyle, butényle, pentényle, ...), des groupes alkynyles (éthynyle, propynyle, butynyle, pentynyle, ...), des groupes cycloalkyles (cyclopentyle, cyclohexyle, ...), des hétérocycles (pipérazine, ...), des groupes aryles (phényle, crésyle, ...), des groupes hétéroaryles (pyridine, pyrimidine, pyrazine, ...), des groupes polyéthylèneglycols ou encore des polyamines.

Les formules (a) et (b) représentent les restes des acides quinique et shikimique et de certains de leurs dérivés, obtenus par protection des hydroxyles portés aux positions 1, 3, 4 et/ou 5 du cycle.

Les composés répondant aux formules générales (a) et (b), lorsqu'ils présentent au moins deux fonctions hydroxyles libres vicinales, constituent des mimes du D-mannose : ces composés, et par conséquent le composé de formule générale (II) qui les porte, sont capables d'être reconnus par le récepteur du mannose et de se lier à ce dernier ou à tout récepteur de la famille des C-lectines apparenté au récepteur du mannose.

Le composé A' est avantageusement formé d'un enchaînement comprenant plusieurs restes d'un composé au moins trifonctionnel, liés les uns aux autres par des liaisons covalentes, et propre à offrir, au niveau des groupes fonctionnels libres de ces restes (c'est-à-dire des groupes fonctionnels qui ne sont pas engagés dans une liaison covalente), plusieurs sites d'ancrage pour le ou les composés de formule générale (a) et/ou (b) ou, lorsque X est présent, le composé X'.

Le composé A' comprend de préférence de 2 à 32 et, de manière encore préférée, de 4 à 16 restes d'un composé trifonctionnel.

De préférence, le composé A' comprend un enchaînement d'acides aminés, identiques ou différents, sélectionnés dans le groupe constitué par la lysine, l'hydroxylysine, la sérine, la thréonine, la cystéine, l'ornithine, l'acide aspartique et l'acide glutamique. Cet acide aminé peut être aussi bien de la série L que de la série D et le composé A' peut même comprendre à la fois des résidus de la série L et des résidus de la série D. Un composé A' à base de lysine est préféré, dans la mesure où il a été montré que des macromolécules à base de lysine sont dénuées d'immunogénicité intrinsèque (TAM, Proc. Natl. Acad. Sci. USA, 1988, 85, 540).

Toutefois, il est possible d'utiliser, en tant que composé A', des enchaînements de composés trifonctionnels autres que les acides aminés cités ci-dessus, pour autant qu'ils présentent une biocompatibilité satisfaisante. A titre d'exemples et de façon non-limitative, on peut citer la 3,3'-iminobis(propylamine), la 2,2'-iminobis(éthylamine), l'acide gallique, le tris(2-carboxyéthyl)nitrométhane (M. BRETTEICH et al., Synlett, 1998, 1396), le tris(hydroxyméthyl)aminométhane (P. R. ASHTON et al., J. Org. Chem., 1998, 63, 3429) et la (dihydroxy)propylamine.

L'indice n, qui représente le nombre de restes X présents dans le composé de formule générale (II), peut être :
- soit égal à 0, auquel cas le ou les restes B² sont directement liés au reste A,
- soit compris entre 1 et 32, auquel cas, si n est égal à 1, le ou les restes B² sont liés au reste A par l'intermédiaire d'un seul reste X, tandis que, si n est différent de 1, chaque reste X peut servir d'élément de liaison entre un ou plusieurs restes B² et le reste A.

Quant aux groupes de liaison M et N, ils sont choisis, indépendamment les uns des autres, parmi les groupes de liaison qui comprennent une fonction choisie parmi les fonctions oxime, hydrazone, amide, ester, thioester, hydrazide, hydroxamate, éther, thioéther, amine, carbonate, carbamate, thiocarbonate, thiocarbamate, urée, thiourée et thiazolidine. A titre d'exemples et de façon non-limitative :
- une liaison oxime peut être formée par réaction d'un groupe O-alkylamine avec un groupe carbonyle,
- une liaison hydrazone peut être formée par réaction d'un groupe hydrazine avec un groupe carbonyle,
- une liaison amide (respectivement, ester) peut être formée par réaction d'un groupe amine (respectivement, alcool) avec un groupe acide activé ou anhydride ou halogénure d'acide,
- une liaison thioester peut être formée par réaction d'un groupe thiol avec un groupe acide activé ou anhydride ou halogénure d'acide,
- une liaison hydrazide peut être formée par réaction d'un groupe hydrazine avec un groupe acide activé, anhydride ou halogénure d'acide,
- une liaison éther peut être formée par réaction d'un groupe alcool avec un groupe halogénure d'alkyle,
- une liaison thioéther peut être formée par réaction d'un thiol avec un groupe halogénure d'alkyle,
- une liaison amine peut être formée par réaction entre un groupe amine et un halogénure d'alkyle,
- une liaison carbonate peut être formée par réaction d'un groupe chloroformate avec un groupe alcool,
- une liaison carbamate peut être formée par réaction d'un groupe chloroformate avec un groupe amine,
- une liaison thiocarbonate peut être formée par réaction d'un groupe chlorothioformate avec un groupe alcool,
- une liaison thiocarbamate peut être formée par réaction d'un groupe chlorothioformate avec un groupe amine,
- une liaison urée peut être formée par réaction d'un groupe isocyanate avec un groupe amine,
- une liaison thiourée peut être formée par réaction d'un groupe isothiocyanate avec un groupe amine,
- une liaison thiazolidine peut être formée par réaction d'un groupe β-aminothiol avec un groupe carbonyle, tandis que
- une liaison hydroxamate peut être formée par réaction entre un groupe acide activé et un groupe hydroxylamine.

On comprendra dès lors que, dans la formule générale (II), B², A et X représentent les restes de composés portant naturellement des groupes fonctionnels propres à conduire, par réaction les uns sur les autres, à l'obtention des groupes de liaison M et N tels que définis ci-dessus, ou de composés ayant été modifiés de manière à porter de tels groupes fonctionnels.

Le composé de formule générale (II) est avantageusement tel que m est un nombre entier compris entre 4 et 16, n est un nombre entier compris entre 2 et 8, X représente le reste d'un oligopeptide comprenant de 2 à 4 résidus d'acides aminés, tandis que A représente le reste d'un enchaînement comprenant de 2 à 8 résidus de lysine et se présentant sous la forme d'un dendrimère.

Les composés de formules générales (a) et/ou (b) sont avantageusement choisis de telle sorte que les groupes -OR₂ et -OR₃ soient en relation trans, de manière à mimer les deux hydroxyles portés par le cycle du D-mannose qui apparaissent être impliquées dans la liaison entre ce composé et son récepteur.

Le composé de formule (b) est avantageusement tel que R₁, R₂ et R₃ représentent des atomes d'hydrogène. Si, en outre, les groupes -OR₁ et -OR₂ se trouvent en relation cis, et les groupes -OR₂ et -OR₃ se trouvent en relation trans, la configuration étant 3R, 4S et 5R, le composé résultant consiste en un reste de l'acide (-)-shikimique (ou acide 3,4,5-trihydroxy-1-cyclohexène-1-carboxylique).

Quant au composé de formule (a), il est avantageusement tel que R₁, R₂, R₃ et R₄ représentent des atomes d'hydrogène. Si, en outre, les groupes -OR₂ et -OR₃ se trouvent en relation trans, alors que les groupes -OR₁ et -OR₄ se trouvent chacun en relation cis par rapport au groupe -OR₂, la configuration étant 1sₙ, 3R, 4sₙ et 5R, alors le composé résultant consiste en un reste de l'acide (-)-quinique (ou acide 1,3,4,5-tétrahydroxy-cyclohexane-carboxylique).

Ainsi, le composé de formule générale (II) est de préférence tel que B² représente un reste d'un ou plusieurs composés choisis parmi l'acide (-)-shikimique et l'acide (-)-quinique, éventuellement sous forme protégée. Les acides (-)-shikimique et (-)-quinique sont disponibles commercialement, par exemple auprès des Sociétés ALDRICH ou ACROS.

Le procédé de couplage conforme à l'invention peut mettre en oeuvre plusieurs composés peptidiques différents et/ou plusieurs vecteurs lipophiles différents, permettant ainsi de synthétiser des mélanges de composés peptidiques fonctionnalisés par des vecteurs lipophiles.

De tels mélanges sont obtenus en une fois à l'issue de l'étape b) du procédé selon l'invention : l'utilisation de plusieurs composés peptidiques différents et/ou de plusieurs vecteurs lipophiles différents lors de l'étape b) aboutit directement à un mélange de composés peptidiques fonctionnalisés par des vecteurs lipophiles, mélange qui est directement utilisable, sans étape de purification préalable ni de dessalage, par lyophilisation directe du milieu réactionnel ou précipitation des composés obtenus par dilution à l'eau.

Selon un mode de mise en oeuvre avantageux du procédé de couplage conforme à l'invention, ce dernier met en oeuvre, lors de l'étape b), au moins un vecteur lipophile portant une fonction aldéhyde, au moins un composé peptidique choisi parmi les peptides, les glycopeptides et les pseudopeptides, ainsi qu'au moins un glycomimétique dendrimérique de formule générale (II) telle que définie ci-dessus.

L'utilisation d'au moins un composé de formule (II) au cours de l'étape b) du procédé de couplage selon l'invention aboutit avantageusement à l'obtention d'un mélange comprenant au moins un composé peptidique choisi parmi les peptides, les glycopeptides et les pseudopeptides, et au moins un glycomimétique dendrimérique de formule (II), chacun étant respectivement lié à un ou plusieurs vecteurs lipophiles. De par la présence des chaînes lipophiles, ledit mélange s'organise sous la forme de micelles mixtes. La présence du composé de formule (II) dans ces micelles permet de vectoriser sélectivement lesdits composés peptidiques vers les cellules possédant des récepteurs du mannose ou des récepteurs de la famille des C-lectines apparentés au récepteur du mannose.

L'invention a également pour objet un vecteur lipophile utilisable dans le procédé de couplage défini ci-avant, caractérisé en ce qu'il répond à la formule générale (I) :

[(R¹)(R²)ᵢ] D-CHO (I)

telle que définie précédemment.

Un tel vecteur lipophile est avantageusement tel que i est égal à 1 et D représente l'hétérocycle 1-aza-3,7-dioxabicyclo (3.3.0)-octane.

Le groupement L (dans la formule L-f-E-f' définie en rapport avec R¹ et R²) représente par exemple un stérol, un dérivé de stérol (tel qu'un dérivé du cholestérol) ou une chaîne carbonée saturée ou insaturée, linéaire ou ramifiée, comprenant entre 4 et 30 atomes de carbone. Une telle chaîne carbonée constitue la partie lipophile d'un acide gras, tel que l'acide palmitique (lorsque L répond à la formule CH₃-(CH₂)₁₄-) ou l'acide oléique (lorsque L répond à la formule CH₃-(CH₂)₇-CH=CH-(CH₂)₇-).

Par ailleurs, E représente avantageusement une chaîne carbonée linéaire, ramifiée ou cyclique, saturée ou insaturée, comportant de 1 à 18 atomes de carbone et, éventuellement, 1 à 16 hétéroatomes (par exemple de l'azote ou de l'oxygène) et/ou 1 à 7 groupements sélectionnés parmi les groupements carbonyles, les hétérocycles, les hétéroaryles, les carbocycles et les aryles. E peut éventuellement être substitué par 1 à 8 groupements hydroxyles ou amino et/ou 1 à 16 atomes d'halogène (tels que le chlore ou le fluor).

Au sens de la présente invention, on entend par :
- « carbocycle » (aussi appelé « cycloalkyle ») un cycle carboné mono- ou polycyclique, comprenant entre 3 et 8 atomes de carbone, tel que le cyclopentyle ou le cyclohexyle ;
- « aryle » un cycle carboné aromatique, mono- ou polycyclique, comprenant entre 5 et 14 atomes de carbone, tel que le phényle, le naphtyle ou le crésyle ;
- « hétérocycle » un carbocycle comprenant un ou plusieurs hétéroatomes choisis parmi l'azote, l'oxygène et le soufre, tel que la pipérazine ;
- « hétéroaryle » un aryle comprenant un ou plusieurs hétéroatomes choisis parmi l'azote, l'oxygène et le soufre, tel que la pyridine, la pyrimidine ou la pyrazine.

Quant à f et f, ces fonctions représentent par exemple, respectivement, une liaison -CO-NH- ou -CO-O- et -NH-CO- ou -O-CO-.

Des exemples de vecteurs lipophiles préférés conformes à l'invention répondent aux formules (III) et (IV) ci-après : 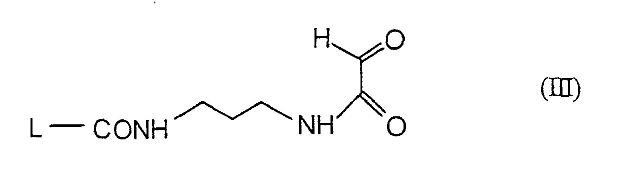 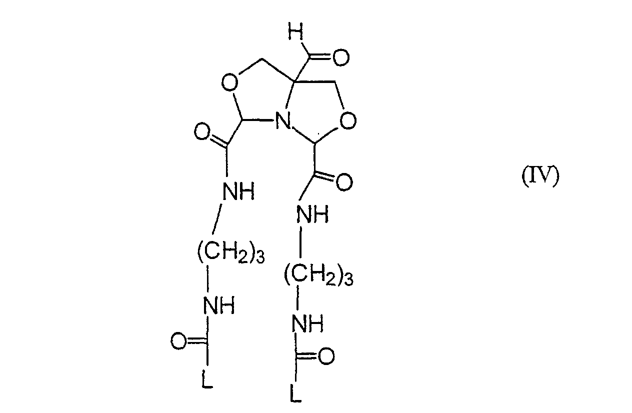 dans lesquelles L est tel que défini précédemment et représente, par exemple :
- dans les formules III et IV, une chaîne carbonée de formule CH₃-(CH₂)₁₄- (c'est-à-dire la partie lipophile de l'acide palmitique), ou une chaîne carbonée de formule CH₃-( CH₂)₇-CH=CH-(CH₂)₇- (c'est-à-dire la partie lipophile de l'acide oléique).
- dans la formule III, une chaîne carbonée de formule CH₃-(CH₂)₇-CH=CH-(CH₂)₇- (c'est-à-dire la partie lipophile de l'acide oléique), ou un dérivé de stérol.

Les vecteurs lipophiles conformes à l'invention sont particulièrement adaptés pour être utilisés, tels quels et sans qu'une activation préalable soit nécessaire, dans le procédé de couplage défini précédemment.

L'invention a également pour objet un lipopeptide susceptible d'être obtenu par le procédé de couplage tel que défini ci-avant, caractérisé en ce qu'il est essentiellement constitué d'au moins un composé peptidique lié, par un lien hydrazone, à au moins un vecteur lipophile de nature non peptidique de formule (I) tel que défini ci-avant. Ledit composé peptidique est avantageusement tel que défini précédemment en rapport avec le procédé de couplage conforme à l'invention.

Comme cela a été évoqué précédemment, un tel lipopeptide possède la capacité de pénétrer dans les cellules par transfert passif à travers la membrane cellulaire, de façon non sélective.

L'invention a également pour objet un mélange de plusieurs lipopeptides différents tels que définis ci-dessus. Un tel mélange est susceptible d'être obtenu par le procédé de couplage conforme à l'invention tel que précédemment décrit.

L'invention a également pour objet l'utilisation d'un lipopeptide tel que défini ci-dessus pour le ciblage cellulaire.

L'invention a également pour objet l'utilisation du procédé de couplage défini ci-dessus pour la préparation d'un médicament comprenant au moins un principe actif de nature peptidique vectorisé par au moins un composé lipophile, utile pour le ciblage cellulaire.

Dans un tel médicament, le principe actif de nature peptidique (par exemple une hormone ou un neuropeptide), vectorisé par un composé lipophile, est apte à traverser les barrières physiologiques lipophiles, telles que les membranes cellulaires, et ce de façon non sélective (c'est-à-dire quelles que soient les cellules). Les propriétés physiques du principe actif se trouvent modifiées, puisque ce dernier, de par la présence de chaînes lipophiles, se présente sous la forme de micelles ou d'agrégats, qui permettent notamment, dans le cas de micelles mixtes, de regrouper des peptides de natures différentes dans un même environnement. Lorsque ces micelles ou agrégats comprennent également un composé de formule (II) lié, par un lien hydrazone, à au moins un vecteur lipophile, le médicament résultant permet de cibler les récepteurs de la famille des C-lectines apparentés au récepteur du mannose.

Outre les dispositions qui précèdent, l'invention comprend encore d'autres dispositions qui ressortiront de la description qui va suivre, qui se réfère à des exemples de mise en oeuvre du procédé objet de la présente invention et de synthèses de lipopeptides selon la présente invention, ainsi qu'aux dessins annexés, dans lesquels :
- la figure 1 illustre la synthèse des vecteurs lipophiles de formules (IIIa) et (IIIb) et (IIIc) conformes à l'invention ;
- la figure 2 illustre une autre voie de synthèse du vecteur lipophile de formule (IIIa), ainsi que la synthèse du vecteur lipophile de formule (IVa) conforme à l'invention ;
- la figure 3 illustre la synthèse du vecteur lipophile de formule (IVb) conforme à l'invention ;
- la figure 4 illustre le couplage, par le procédé conforme à l'invention, d'un mélange de peptides dénommés SIV1-7 et TT avec un vecteur lipophile de formule (IIIa) ;
- la figure 5 illustre le couplage, par le procédé conforme à l'invention, du peptide dénommé Mu-IFNγ avec deux vecteurs lipophiles de formule (IIIa) ;
- la figure 6 illustre le couplage, par le procédé conforme à l'invention, du peptide dénommé OVA avec un vecteur lipophile de formule (IVa).

Il doit être bien entendu, toutefois, que ces exemples sont donnés uniquement à titre d'illustration de l'objet de l'invention, dont ils ne constituent en aucune manière une limitation.

Dans les exemples qui suivent, les abréviations suivantes sont utilisées : eq. : équivalents ; Boc : tert-butyloxycarbonyle ; Fmoc : 9-fluorènylméthoxycarbonyle ; Mtt : 4-méthyl-trityle ; DMF : diméthylformamide ; TFA : acide trifluoroacétique ; CH₂Cl₂ : dichlorométhane ; MeOH : méthanol ; EtOH : éthanol ; THF : tétrahydrofurane ; AcOH : acide acétique ; AcOEt : acétate d'éthyle ; H₂O : eau ; AcO⁻NH₄⁺ : acétate d'ammonium ; CDCl₃ : chloroforme deutéré ; EDT : éthanedithiol ; TIS : triisopropylsilane ; DMSO-d6 : diméthylsulfoxyde-d6 (entièrement deutéré) ; NaIO₄ : periodate de sodium ; NaCl : chlorure de sodium ; Na₂SO₄ : sulfate de sodium ; MgSO₄ : sulfate de magnésium ; KH₂PO₄ : dihydrogénophosphate de potassium ; PAL : « peptide-amide linker»; BOP : benzotriazole-1-yl-oxy-tris(diméthylamino)phosphoniumhexafluorophosphate ; HOBt : N-hydroxybenzotriazole; HBTU: N-oxyde d'hexafluorophosphate de N-[(1H-benzotriazol-1-yl)(diméthylamino)méthylène]-N-méthylméthanaminium ; DIEA : diisopropyléthylamine ; TEAP : phosphate de triéthylamine ; HPLC : chromatographie liquide haute performance ; RP-HPLC : chromatographie liquide haute performance en phase inverse ; ES-MS : spectrométrie de masse par électronébulisation ; TOF-PDMS : spectrométrie de masse à désorption par plasma ; LC-MS : spectrométrie de masse couplée à une analyse par chromatographie liquide ; RMN ¹H : résonance magnétique nucléaire du proton ; RMN ¹³C : résonance magnétique nucléaire du carbone. PyBOP : benzotriazole-1-yl-oxy-tris-pyrrolidinophosphonium hexafluorophosphate.

### EXEMPLE 1 : Synthèse de peptides fonctionnalisés par des groupements α-hydrazinoacétiques

Les peptides suivants, fonctionnalisés par des groupements α-hydrazinoacétiques soit au niveau de leur extrémité N-terminale, soit au niveau de l'extrémité de la chaîne latérale d'une lysine (K) présente dans la séquence peptidique, ont été préparés :
SIV1 : H-SVRPKVPLRAMTYKLAIDMSHFIKEKK(COCH₂NHNH₂)-NH₂
SIV2 : H-EKGGLEGIYYSARRHRILDMYLEK(COCH₂NHNH₂)-NH₂
SIV3 : H-DWQDYTSGPGIRYPKTFGWLWKLVK(COCH₂NHNH₂)-NH₂
SIV4 : H-SKWDDPWGEVLAWKFDPTLAYTYEAK(COCH₂NHNH₂)-NH₂
SIV5 : H-YTYEAYARYPEELEASQACQRKRLEEGK(COCH₂NHNH₂)-NH₂
SIV6 : H-KFGAEVVPGFQALSEGCTPYDINQMLNCVGDK(COCH₂NHNH₂)-NH₂
SIV7 : H-QIQWMYRQQNPIPVGNIYRRWIQLGLQKCVRMYNPTN-K(COCH₂NHNH₂)-NH₂
TT : Ac-QYIKANSKFIGITELKK K(COCH₂NHNH₂)-NH₂
Mu-IFNγ : H₂NNHCH₂CO-K(COCH₂NHNH₂)-AKFEVNNPQVQRQAFNELIR-VVHQLLPESSLRKRKRSR-NH₂
Mu-IFNγ a : H-K(COCH₂NHNH₂)IRVVHQLLPESSLRKRKRSR-NH₂
Mu-IFNγ b : H-K(COCH₂NHNH₂)SLRSERRHQKVRPIRVLSKL-NH₂
Mu-IFNγ c : **H-K**(COCH₂NHNH₂)-
AKFEVNNPQVQRQAFNELIRVVHQLLPESSLRKRKRSR-NH₂
Mu-IFNγ d : H-K(COCH₂NHNH₂)-
PSRENQNAVKIQKLSVVLRREQKHRVERLAFRNQSLPF-NH₂
OVA : Ac-K(COCH₂NHNH₂)-ISQAVHAAHAEINEAGR-NH₂
G18R : H-K(COCH₂NHNH₂)GAWGGLGGYMLGSAMSR-NH₂

La synthèse de ces hydrazinopeptides s'effectue en phase solide selon les protocoles décrits ci-après.

### 1) Préparation de la résine Fmoc-Lys(Boc₂NN(Boc)CH₂CO)-PAL-PEG-PS.

La résine de départ est la résine Fmoc-PAL-PEG-PS de charge 0,16 mmol/g (Perseptive Biosystems, 25 g). Elle est traitée avec de la pipéridine à 20% dans le DMF pour déprotéger la fonction amine, puis lavée à l'éthanol, à la N-méthylpyrrolidone et au DMF. L'acide aminé Fmoc-Lys(Mtt)-OH (Senn Chemicals), utilisé à raison de 4 eq. par rapport aux fonctions amines réactives présentes sur le support solide, est activé par le mélange HBTU/HOBt/DIEA (4 eq./4 eq./8 eq.) dans le DMF pendant 1 minute. L'acide activé est ensuite ajouté à la résine. Après 1 h d'agitation, la résine est lavée au DMF, puis au CH₂Cl₂ et stockée dans du CH₂Cl₂ à 4°C.

La déprotection du groupement Mtt est réalisée par une série de lavages avec du TFA à 1% dans le CH₂Cl₂. Cette déprotection est suivie par RP-HPLC sur colonne TSK gel Super-ODS (Tosohaas). La déprotection est achevée après 13 à 16 lavages. La résine est neutralisée par deux lavages à la diisopropyléthylamine à 5% dans le CH₂Cl₂, puis lavée avec du CH₂Cl₂.

Le couplage de l'acide N,N'-tri(Boc)hydrazinoacétique, réalisé comme décrit dans la Demande internationale PCT/FR00/02336, est effectué en utilisant 1,2 eq. d'acide N,N'-tri(Boc)hydrazinoacétique par rapport aux fonctions amines réactives présentes sur le support solide. L'acide N,N'-tri(Boc)hydrazinoacétique est activé à l'aide d'un mélange BOP/DIEA (M. GAIRI et al., Tetrahedron Letters, 1990, 50, 7363) ou HBTU/HOBt/DIEA dans le DMF (acide N,N'-tri(Boc)-hydrazino-acétique/BOP/DIEA : 1/1/3, ou acide N,N'-tri(Boc)hydrazino-acétique/HBTU/HOBt/ DIEA : 1/1/1/3), puis ajouté en une fois au support solide en suspension dans le DMF. Après 30 minutes, le support est lavé par du DMF et du CH₂Cl₂.

La résine Fmoc-Lys(Boc₂NN(Boc)CH₂CO)-PAL-PEG-PS ainsi préparée est utilisée directement pour les synthèses peptidiques sur support.

### 2) Synthèse des peptides SIV1 à SIV7 et TT.

### • Protocole de synthèse.

Les hydrazinopeptides SIV1-7 et TT sont synthétisés en phase solide sur la résine Fmoc-Lys(Boc₂NN(Boc)CH₂CO)-PAL-PEG-PS en utilisant la chimie Fmoc/tert-butyle, telle que décrite par exemple dans « Fmoc solid phase peptide synthesis, a practical approach » (W.C. CHAN & P.D. WHITE Editors, Oxford Iuniversity Press, Oxford 2000) ou par G.B. FIELDS et al. dans Int. J. Pept. Protein, 1990, 35, 161, avec une activation HBTU/HOBt (M. SCHNÖLZER et al., Int. J. Pept. Protein Res., 1992, 40, 180). La synthèse est réalisée sur un synthétiseur automatique Pioneer (Perseptive Biosystems). Les réactifs de synthèse sont les suivants :
- déprotection des fonctions amines : pipéridine à 20% dans le DMF,
- activateur des acides aminés : HBTU/HOBt (les deux réactifs étant utilisés à la concentration de 0,5 M dans le DMF),
- solution de DIEA 1M dans le DMF,
- solution de « capping » (c'est-à-dire de blocage des fonctions réactives qui n'ont pas réagi) : anhydride acétique à 3% et DIEA 0,3% dans le DMF.

Les hydrazinopeptides SIV1 et SIV2 ont été synthétisés sur une échelle de 0,1 mmole, en utilisant 10 équivalents en acides aminés. Quant aux peptides SIV3 à SIV7 et TT, ils ont été synthétisés sur une échelle de 0,25 mmole en utilisant 4 équivalents en acide aminés. Les synthèses ont été réalisées en double couplage avec « capping », selon la succession d'étapes suivante : déprotection de la fonction amine, activation de l'acide aminé et couplage (deux fois), puis « capping » avec la solution d'anhydride acétique.

### • Coupure et déprotection des hydrazinopeptides SIV1-7 et TT.

Les hydrazinopeptides sont coupés du support solide, et simultanément déprotégés, par l'action du TFA en présence de piégeurs de carbocations. Le mélange utilisé est le suivant :
- pour les peptides SIV1 et SIV2 : TFA 95%, TIS 2,5%, H₂O 2,5%,
- pour le peptide SIV4 : TFA 95%, EDT 2,5%, H₂O 2,5%,
- pour les peptides SIV3, SIV5, SIV6, SIV7 et TT : TFA/EDT/thioanisole/ H₂O/phénol (10 ml/0,25 ml/0,5 ml/0,5 ml/0,75 g).

La durée de la réaction de coupure des hydrazinopeptides du support solide varie entre 1h30 et 2h30 sous agitation. Les peptides sont ensuite précipités dans l'éther éthylique froid et centrifugés.

### • Purification des hydrazinopeptides SIV1-7 et TT.

Les hydrazinopeptides sont purifiés par RP-HPLC sur une colonne préparative (diamètre de 15 mm et longueur de 500 mm) avec une phase stationnaire C3 (Zorbax) sur une chaîne HPLC Shimadzu 6A équipée d'une table traçante Kipp&Zonen et d'un collecteur de fraction Gilson. Les solvants d'élution (solvants A et B) sont les suivants :
- solvant A : eau desionisée comprenant 0,05% de TFA,
- solvant B : isopropanol à 40% dans de l'eau desionisée comprenant 0,05% de TFA (pour les peptides SIV4 et SIV5), ou n-propanol à 40% dans de l'eau desionisée comprenant 0,05% de TFA (pour les autres peptides).

Les hydrazinopeptides bruts sont mis en solution dans un mélange eau/acide acétique à hauteur de 10 à 20 mg/ml pour des quantités injectées allant de 40 à 80 mg, selon le peptide. Les gradients de purification varient en fonction des hydrazinopeptides injectés :
SIV1 : de 15 à 40% de tampon B en 25 minutes (4 ml/min, 50°C),
SIV2 : de 15 à 30% de tampon B en 30 minutes (3 ml/min, 50°C),
SIV3 : de 20 à 40% de tampon B en 20 minutes (3 ml/min, 50°C),
SIV4 : de 20 à 50% de tampon B en 30 minutes (3 ml/min, 50°C),
SIV5 : de 15 à 30% de tampon B en 15 minutes (3 ml/min, 50°C),
SIV6 : de 30 à 60% de tampon B en 25 minutes (3 ml/min, 50°C),
SIV7 : de 30 à 40% de tampon B en 30 minutes (3 ml/min, 50°C),
TT : de 0 à 25% de tampon B en 25 minutes (3 ml/min, 50°C).

La détection UV se fait à 225 nm, sauf dans le cas de SIV7 pour lequel la détection se fait à 280 nm. Les fractions récoltées sont analysées par RP-HPLC (Chaîne Shimadzu 10A) sur colonne C3 (Zorbax, diamètre de 4,6 mm et longueur de 160 mm) avec un gradient linéaire de 0 à 100% du tampon B en 30 minutes (détection à 215 nm, débit de 1 ml/min, 50°C). Les fractions contenant les hydrazinopeptides sont réunies et lyophilisées.

Le tableau I rassemble, pour les hydrazinopeptides SIV1 à SIV7 et TT, les masses de peptides bruts obtenues après la synthèse, les masses de peptides purifiés obtenues après purification par RP-HPLC, ainsi que le pourcentage de pureté de ces peptides purifiés.

**Tableau I**

| | Peptide brut (mg) | Peptide purifié (mg) | Pureté (%) |
|---|---|---|---|
| SIV1 | 284 | 35 | 100 |
| SIV2 | 251 | 42 | 93 |
| SIV3 | 365 | 68 | 90 |
| SIV4 | 532 | 141 | 99 |
| SIV5 | 661 | 48 | 93 |
| SIV6 | 637 | 148 | 98 |
| SIV7 | 776 | 68 | 82 |
| TT | 434 | 120 | 97 |

### • Analyse des hydrazinopeptides SIV1-7 et TT.

Afin de confirmer leur identité, les hydrazinopeptides purifiés sont analysés par ES-MS à l'aide d'un spectromètre Micromass Quatro. Les poids moléculaires calculés (« calc. ») et observés (« obs. ») sont les suivants : SIV1 calc. 3258.9, obs. 3257.5 ; SIV2 calc. 2969.35, obs. 2968 ; SIV3 calc. 3113.5, obs. 3111.5 ; SIV4 calc. 3188.5, obs. 3187 ; SIV5 calc. 3453.7, obs. 3452.5 ; SIV6 calc. 3502.9, obs. 3501.5 ; SIV7 calc.4806.5, obs. 4804.5 ; TT calc. 2222.57, obs. 2220.5.

Les hydrazinopeptides sont également analysés par électrophorèse capillaire (Applied Biosystems 270A-HT). Les conditions opératoires sont les suivantes :
- SIV4, SIV5 et SIV6 : tampon borate 50 mM, 30 kV, 10 minutes, 200 nm, 45°C, injection pendant 1,5 secondes,
- pour les autres hydrazinopeptides : tampon acide citrique 20 mM, pH 2,1, 30 kV, 10 minutes, 200 nm, 45°C, injection pendant 1,5 secondes.

Les hydrazinopeptides sont soumis, en outre, à une analyse de la composition en acides aminés après hydrolyse acide totale avec de l'acide chlorhydrique 6N, pendant 24 heures et à 110°C. Outre une troisième confirmation de leur identité, cette analyse permet d'estimer le pourcentage de peptide dans le lyophilisat. Les résultats de cette analyse sont les suivants : SIV1 : 91.9% ; SIV2 : 78.2% ; SIV3 : 97.9% ; SIV4 : 100%; SIV5 : 92.7% ; SIV6 : 74.9% ; SIV7 : 95.1% ; TT : 100%.

### 3) Synthèse des peptides Mu-IFNγ, Mu-IFNγ a, Mu-IFNγ b, Mu-IFNy c et Mu-IFNγ d.

Les séquences présentes dans les peptides Mu-IFNγ et Mu-IFNγ c, d'une part, et Mu-IFNγ a, d'autre part, correspondent respectivement aux 38 et aux 20 acides aminés contigus de l'extrémité C-terminale de l'interféron-γ (IFN-γ) murin, comme décrit notamment dans la Demande internationale. PCT publiée sous le numéro WO 99/40113, et par K. THIAM et al. dans Biochemical and Biophysical Research Communications, 1998, 253, 639-647 et dans Journal of Medicinal Chemistry, 1999, 42, 3732-3736. Quant aux séquences présentes dans les peptides Mu-IFNγ b et Mu-IFNγ d, il s'agit de séquences « scramble » de celles présentes dans les peptides Mu-IFNγ a et Mu-IFNγ c, respectivement, c'est-à-dire de séquences dans lesquelles l'ordre des acides aminés a été disposé de façon à éviter toute parenté séquentielle avec le peptide d'origine. Un tel peptide servira de contrôle dans les tests biologiques qui seront décrits ci-après.

### • Synthèse de l'hydrazinopeptide Mu-IFNγ.

L'hydrazinopeptide Mu-IFNγ est préparé à l'aide de 0,25 mmole d'une résine Rink amide MBHA PS de charge 0,74 mmol/g (Applied Biosystems, Foster City, USA), selon la stratégie Fmoc/tert-butyle (comme décrit ci-dessus pour la synthèse des hydrazinopeptides SIV1 à SIV7 et TT) et une activation classique sur un synthétiseur de peptides Applied Biosystems 430 A. En fin de synthèse, une partie de la peptidyl-résine (31,25 µmoles) est modifiée à l'extrémité N-terminale par un groupement Fmoc-L-Lys(Fmoc)-OH. Après déplacement des groupements Fmoc par une solution de pipéridine à 20% dans le DMF, l'acide N,N'-tri(Boc)hydrazinoacétique (36,48 mg, 1,2 eq. par groupe amino) est introduit manuellement en utilisant l'activation BOP in situ, comme décrit en 1) ci-dessus.

La coupure et la déprotection de l'hydrazinopeptide Mu-IFNγ est réalisée en présence d'un mélange TFA/H₂O/phénol/EDT/thioanisole (1 g de résine sèche / 10,0 ml de TFA / 0,5 ml d'eau / 0,75 g de phénol / 0,25 ml d'EDT / 0,5 ml de thioanisole). Après précipitation dans un mélange éther/heptane (1/1 en volume) et centrifugation, le peptide est repris dans un mélange eau/acide acétique (80/20 en volume), congelé et lyophilisé.

L'hydrazinopeptide Mu-IFNγ est purifié par RP-HPLC sur une colonne préparative (diamètre de 15 mm et longueur de 300 mm) avec une phase stationnaire C3 (Zorbax), comme décrit pour la purification des hydrazinopeptides SV1 à SV7 et TT. Le débit d'élution est de 3 ml/minute en utilisant un gradient de 25 à 90% du solvant B (isopropanol à 40% dans de l'eau desionisée comprenant 0,05% de TFA) dans le solvant A (eau desionisée comprenant 0,05% de TFA). Après lyophilisation, l'hydrazinopeptide Mu-IFNγ (16,1 mg, rendement de 8,5%) est stocké à -20°C. Son analyse ES-MS est la suivante : [M+H]⁺ calculé 4846,6 ; trouvé 4845,5.

### • Synthèse des hydrazinopeptides Mu-IFNγ a et Mu-IFNγ b.

On procède comme décrit ci-dessus en rapport avec la synthèse de l'hydrazinopeptide Mu-IFNγ. Après les étapes de coupure et de déprotection, les peptides sont précipités dans l'éther éthylique, centrifugés, repris dans un mélange eau/acide acétique (90/10 en volume), congelés et lyophilisés. On obtient respectivement 430 mg et 594,4 mg de peptides Mu-IFNγ a et Mu-IFNγ b sous forme brute. La purification des peptides s'effectue comme décrit ci-dessus pour le peptide Mu-IFNγ, en utilisant un gradient de 0 à 50% du solvant B en 50 minutes. On obtient ainsi respectivement 61,4 mg et 104,2 mg de peptides Mu-IFNγ a et Mu-IFNγ b purifiés. Mu-IFNya: TOF-PDMS [M+H]⁺ calculé 2659,22 ; trouvé 2660,9. Mu-IFNγ b : ES-MS [M+H]⁺ calculé 2658,22 ; trouvé 2657,0.

### • Synthèse des hydrazinopeptides Mu-IFNγ c et Mu-IFNγ d.

Les hydrazinopeptides Mu-IFNγ c et Mu-IFNγ d sont préparés à l'aide de 0,1 mmole d'une résine Fmoc-PAL-PEG-PS (Perseptive Biosystems), selon la stratégie Fmoc/*tert*-butyle (comme décrit ci-dessus pour la synthèse des hydrazinopeptides SIV1 à SIV7 et TT) et une activation classique sur un synthétiseur de peptides Pioneer (Perseptive Biosystems). Les synthèses sont réalisées en simple couplage et un excès de 10 en acide aminé par rapport à la charge théorique de la peptidyl-résine. En fin de synthèse, les peptidyl-résines sont modifiés à l'extrémité N-terminale par un résidu Boc-L-Lys(Fmoc)-OH. Après déplacement du groupe Fmoc par une solution de pipéridine à 20% dans le DMF, l'acide N,N'-tri(Boc)hydrazinoacétique (390 mg, 10 équivalents) est également introduit automatiquement. Les étapes de coupure et de déprotection sont réalisées comme décrites ci-dessus pour la préparation du Mu-IFNγ. La purification des peptides Mu-IFNγ c et Mu-IFNγ d s'effectue comme décrit ci-dessus pour le peptide Mu-IFNγ, en utilisant, respectivement, un gradient de 25 à 100% de tampon B en 75 minutes et de 20 à 100% de tampon B en 80 minutes.
158 mg (26%) du peptide Mu-IFNγ c sont ainsi obtenus après lyophilisation. ES-MS calculé 4772,6 ; trouvé 4771,5 ; m/z 1193,5 [M+4H]⁴⁺ , 955,1 [M+5H]⁵⁺, 796,0 [M+6H]⁶⁺, 682,2 [M+7H]⁷⁺.
120 mg (20%) du peptide Mu-IFNγ d sont ainsi obtenus après lyophilisation. ES-MS calculé 4772,6 ; trouvé 4771,0 ; m/z 1193,7 [M+4H]⁴⁺, 955,3 [M+5H]⁵⁺, 796,2 [M+6H]⁶⁺, 682,5 [M+7H]⁷⁺.

### 4) Synthèse du peptide OVA.

L'hydrazinopeptide OVA est synthétisé en phase solide à l'aide de 0,10 mmole d'une résine Fmoc-PAL-PEG-PS de charge 0,16 mmol/g (Perseptive Biosystems), en utilisant la chimie Fmoc/tert-butyle et une activation HBTU/HOBt, comme décrit ci-dessus pour la synthèse des hydrazinopeptides SIV1 à SIV7 et TT, sur un synthétiseur de peptides Pioneer. En fin de synthèse, la fonction α-NH₂ de la lysine est traitée pendant 20 minutes en présence de pipéridine à 20% dans le DMF, puis acétylée pendant 10 minutes en utilisant une solution d'anhydride acétique à 10% dans le CH₂Cl₂. Le groupement protecteur Mtt de la fonction ε-NH₂ de la lysine est déplacé à l'aide d'une solution de TFA à 1% dans le CH₂Cl₂. 13 cycles comprenant, à chaque fois, un traitement avec 20 ml de cette solution sont nécessaires. Finalement, la résine est lavée deux fois, pendant 2 minutes, avec 20 ml de CH₂Cl₂.

L'acide N,N'-tri(Boc)hydrazinoacétique (47 mg, 0,12 mmole) est ensuite couplé à la fonction ε-NH₂ de la lysine en utilisant une activation HBTU/HOBt/DIEA (1,12 mmole/0,12 mmole/0,24 mmole) pendant 60 minutes, comme décrit ci-dessus en rapport avec la synthèse des hydrazinopeptides SIV1 à SIV7 et TT. Après avoir lavé et séché la peptidyl-résine, celle-ci est traitée en présence de TFA 95%/H₂O 2,5%/TIS 2,5% (comme décrit ci-dessus pour la coupure et la déprotection des hydrazinopeptides SIV1 et SIV2) pendant 2 heures à température ambiante. L'hydrazinopeptide est enfin précipité, dissous dans un mélange eau/acide acétique, congelé puis lyophilisé.

L'hydrazinopeptide OVA ainsi obtenu sous forme brute est purifié sur une colonne C3 Zorbax. Le débit d'élution est de 4 ml/minute en utilisant un gradient linéaire de 0 à 20%, en 20 minutes, d'un mélange eau desionisée/isopropanol (2/3) comprenant 0,05% de TFA dans un mélange eau desionisée/TFA 0,05%. Après lyophilisation, 74 mg (rendement de 30%) de l'hydrazinopeptide OVA sont obtenus. L'analyse du peptide OVA par ES-MS est la suivante : [M+H]⁺ calculé 2016,1 ; trouvé 2015,0.

### 5) Synthèse du peptide G18R.

La synthèse est effectuée à l'aide d'un synthétiseur automatique Pioneer (Perseptive Biosystems) avec 0,2 mmole de résine Fmoc-PAL-PEG-PS, selon le protocole décrit ci-dessus en rapport avec la synthèse des peptides SIV1-7 et TT. Les étapes de coupure et de déprotection sont effectuées à l'aide d'un mélange TFA/TIS/H₂O/EDT (94%/1%/2 ,5%/2,5% en volumes). Après précipitation dans l'éther éthylique, on obtient 360 mg de peptide brut. Ce peptide est purifié sur une colonne C3 Zorbax, avec un débit d'élution de 4 ml/minute en utilisant un gradient linéaire de 0 à 30%, en 30 minutes, d'un mélange eau desionisée/isopropanol (40% en volume) comprenant 0,05% de TFA dans un mélange eau desionisée/TFA 0,05%. Après lyophilisation, 117 mg de l'hydrazinopeptide G18R sont obtenus. Son analyse par TOF-PDMS est la suivante : [M+H]⁺ calculé 1883,2 ; trouvé 1881,1.

### EXEMPLE 2: Synthèse d'arbre de lysine tétraquinoylé porteur d'un groupement hydrazino, conforme à la formule générale (II) [(Qui)₄AKHdz]

L'arbre (Qui)₄AKHdz est préparé à une échelle de 0,125 mmole à partir d'une résine Rink amide Nleu AM-PS de charge 0,75 mmole/g selon la stratégie Fmoc/*tert*-butyle (comme décrit ci-dessus pour la synthèse des hydrazinopeptides Mu-IFNγ). La synthèse est réalisée manuellement en utilisant un excès de 4 en acide aminé par rapport aux groupements amines réactives présentes sur le support solide et une activation HBTU/HOBt/DIEA 4/4/8 équivalents (M. SCHNÖLZER *et al*., *Int. J*. *Pept*. *Protein Res.,* 1992, 40, 180), en utilisant le DMF comme solvant. Le Fmoc-β-Ala-OH est d'abord couplé à la résine suivi du premier résidu lysine sous la forme du composé Fmoc-L-Lys(Mtt)-OH. Le groupement 4-méthyltrityl (Mtt) est éliminé par traitement avec une solution à 1% d'acide trifluoroacétique dans le dichlorométhane comme décrit dans L. BOUREL *et al*., *J*. *Peptide Sci*., 2000, 6, 264). L'acide N,N'-tri(Boc)hydrazinoacétique est ensuite couplé à la fonction amine de la chaîne latérale (en utilisant un excès de 1,2 équivalent par fonction amine réactive) après pré-activation à l'aide d'un mélange HBTU/HOBt/DIEA 1,2/1,2/2,4 dans le DMF. L'élaboration de l'arbre est poursuivi en couplant une puis deux lysines sous la forme de leurs dérivés Fmoc-L-Lys(Fmoc)-OH. Les deux étapes de couplage sont suivies chacune d'une étape de "capping" (c'est-à-dire du blocage des éventuelles fonctions amines n'ayant pas réagi), par traitement de la peptidyl-résine avec un mélange Ac₂O/DIEA/CH₂Cl₂ (10/5/85) pendant 5 minutes. Après élimination des groupements Fmoc par traitement avec une solution à 20% de pipéridine dans le DMF pendant 20 minutes, la peptidyl-résine est mise en réaction avec l'acide (1sₙ,3R,4sₙ,5R)-1,3,4,5-tétra-acétoxycyclohexane-1-carboxylique (2 équivalents par rapport aux fonctions amines réactives) activé *in situ* par un mélange PyBOP/DIEA (2 et 4 équivalents par rapport aux fonctions amines réactives, respectivement) dans du DMF. Le couplage est renouvelé en utilisant les mêmes réactifs dans une proportion 1/1/2 équivalents par rapport aux fonctions amines réactives initiales. La résine est ensuite lavée par du dichlorométhane (3 x 2 minutes) puis d'éther éthylique (2 x 1 minute) puis séchée.

La peptidyl-résine est ensuite traitée par un mélange TFA/TIS/H₂O (95/2,5/2,5) (6 ml) pendant 1 heure 30 à température ambiante. Le brut de clivage est précipité après filtration dans un mélange refroidi d'éther éthylique/heptane (1 :1) (60 ml). Après centrifugation, le brut est dissous dans un mélange AcOH/H₂O, congelé puis lyophilisé.

Le résidu est repris par une solution aqueuse d'acide acétique à 25% puis purifié par RP-HPLC semi-préparative sur un système Shimadzu LC-4A, en utilisant une colonne Hypersil hyperprep C-18 (300 Å, 8 µm, 10 x 260 mm), à un débit de 3 ml.min⁻¹, à 50°C et une détection à 215 nm : solvant système A : 0.05% TFA dans de l'eau désionisée ; solvant système B : 0.05% TFA dans de l'isopropanol à 40% dans de l'eau désionisée (gradient : de 0 à 30% de tampon B en 30 minutes, puis de 30 à 40% de tampon B en 20 minutes et de 40 à 50% en 50 minutes). Les fractions contenant l'arbre tétra-quinoylé sont réunies, congelées puis lyophilisés. Une partie du résidu (54 mg) est reprise dans un mélange d'hydrazine à 10% dans de l'eau (4 ml), pendant 1 heure à température ambiante pour permettre la déprotection des fonctions alcools des résidus quinoyles. Le milieu réactionnel est ensuite directement purifié dans les conditions décrites précédemment (gradient : de 0 à 20% de tampon B en 40 minutes), pour fournir 15 mg (40%) de l'arbre tétra-quinoylé sous forme de poudre après lyophilisation. ES-MS : *m*/*z* [M+H]⁺ calculé 1369,7 ; trouvé 1369,8 ; [M+Na]⁺ calculé 1391,8 ; trouvé 1391,7.

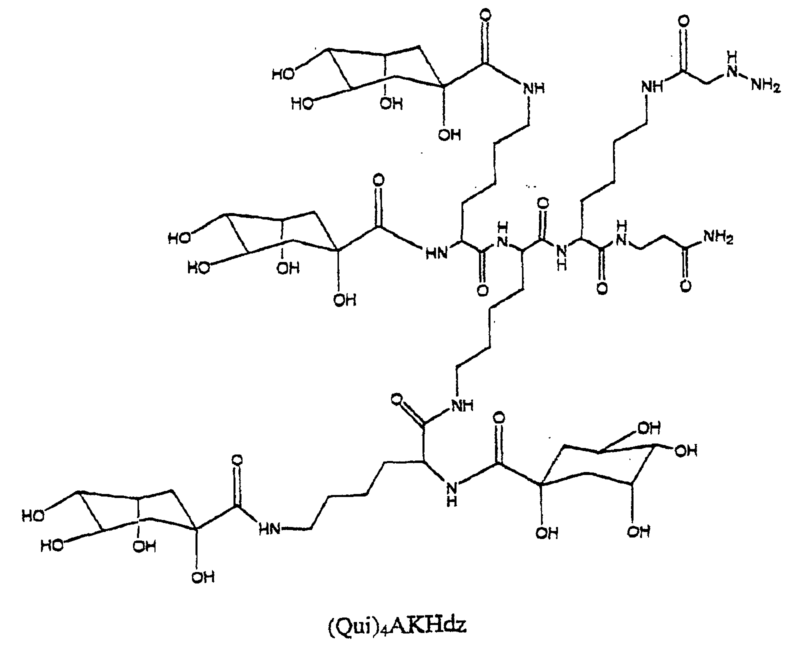

### EXEMPLE 3 : Synthèse de peptides fonctionnalisés par un groupement dérivé d'hydrazine de formule -CO-CHR'-NH-NH₂, où R' H.

Les peptides H₂N-KVGFFKR-NH₂ (R' = 4-aminobutyle) et H₂N-VKYAL-NH₂ (R' = isopropyle) sont synthétisés en phase solide sur une résine Rink amide AM-PS 1%DVD (0,70 mmole/g, 100-200 mesh, Senn Chemicals AG) en utilisant la chimie Fmoc/*tert*-butyle, telle que décrite dans l'EXEMPLE 1, §2, à l'échelle de 0,25 mmole. Les synthèses sont réalisées sur un synthétiseur automatique Applied Biosystems 431A, en simple couplage.

Après avoir complété les séquences peptidiques, les fonctions amines N^{α} de la lysine et de la valine sont déprotégées en utilisant une solution de pipéridine à 20% dans du NMP. Les peptidyl-résines sont ensuite transformées en dérivés d'hydrazines selon une réaction de *N*-amination électrophile en phase solide, utilisant la *N*-Boc-3-(4-cyanophényl)oxaziridine (BCPO), telle que décrite dans la littérature ( D. BONNET *et al*., *J*. *Peptide Res*., 1999, 54, 270 et O. MELNYK *et al*., *J*. *Peptide Res*., 1998, 52, 180).

Brièvement, les peptidyl-résines sont mises en réaction avec 60 mg (0,244 mmole) de BCPO en solution dans CH₂Cl₂ (6 ml) pendant 3 heures. Après lavage par CH₂Cl₂ puis DMF, les résines sont traitées par 50 mg (0,25 mmole) de N benzyl hydrazine sous sa forme chlorhydrate en solution dans DMF/AcOH/H₂O (8,5/1/0,5) (5 ml) (3 x 10 minutes). Après lavage par DMF puis CH₂Cl₂, les peptidyl-résines sont neutralisées par une solution de DIEA à 5% dans CH₂Cl₂ (2 x 2 minutes), et finalement lavées par CH₂Cl₂. Cette série d'opérations est répétée 10 fois.

Les hydrazinopeptides sont déprotégés et clivés de la résine par un traitement avec 10 ml d'un mélange TFA/H₂O/thioanisole/TIS (94/2,5/2,5/1) pendant 1h30. La résine est filtrée sur fritté et les peptides sont précipités dans 100 ml d'un mélange refroidi d'éther éthylique/pentane (1/1). Les résidus sont centrifugés, lyophilisés pour fournir 149 et 133 mg de brut, respectivement, puis purifiés par RP-HPLC semi-préparative sur un système Shimadzu LC-4A, en utilisant une colonne Hypersil hyperprep C-18 (300 Å, 8 µm, 10 x 260 mm), à un débit de 3 ml.min⁻¹, à 50°C et une détection à 215 nm : solvant système A : 0,05% TFA dans de l'eau désionisée ; solvant système B : 0,05% TFA dans de l'isopropanol à 40% dans de l'eau désionisée.

123 mg (36%) du composé H₂N-KVGFFKR-NH₂ sont obtenus après purification (gradient : de 0 à 70% de tampon B en 70 minutes) suivi de la lyophilisation. TOF-PDMS : *m*/*z*[M+H]⁺ calculé 895,9 ; trouvé 895,5.

27,8 mg (13,3%) du composé H₂N-VKYAL-NH₂ sont obtenus après purification (gradient : de 0 à 40% de tampon B en 200 minutes) suivi de la lyophilisation. TOF-PDMS : *m*/*z*[M+H]⁺ calculé 607,8 ; trouvé 607,5.

### EXEMPLE 4 : Synthèse des vecteurs lipophiles (IIIa), (IIIb) (IIIc) et (IVa) conformes à l'invention.

Les vecteurs lipophiles (IIIa) et (IIIb), représentés ci-après, répondent à la formule générale (III) conforme à l'invention, dans laquelle L représente respectivement une chaîne carbonée de formule CH₃-(CH₂)₁₄- ou de formule CH₃-(CH₂)₇-CH=CH-(CH₂)₇-.

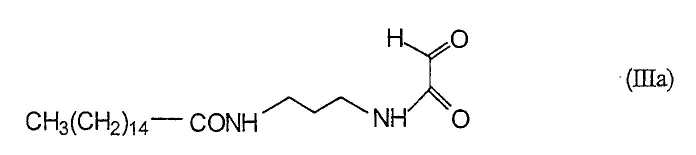

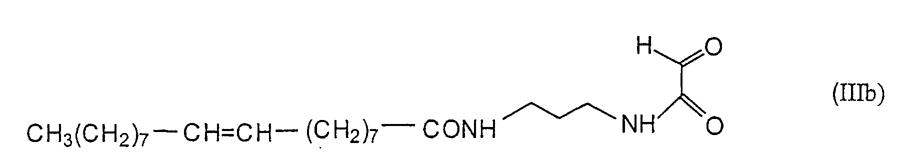

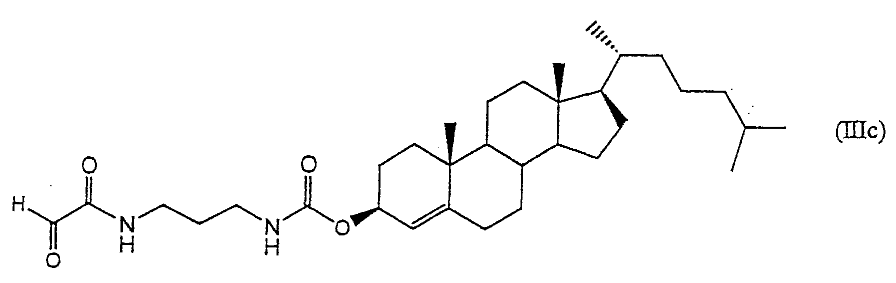

Le vecteur lipophile (IVa), représenté ci-après, répond à la formule générale (IV) conforme à l'invention, dans laquelle L représente une chaîne carbonée de formule CH₃-(CH₂)₁₄-.

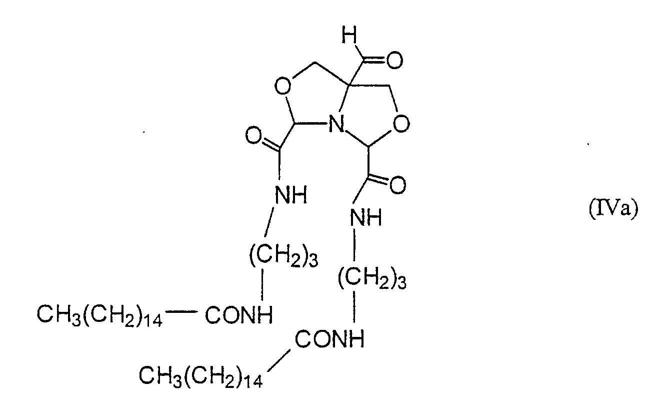

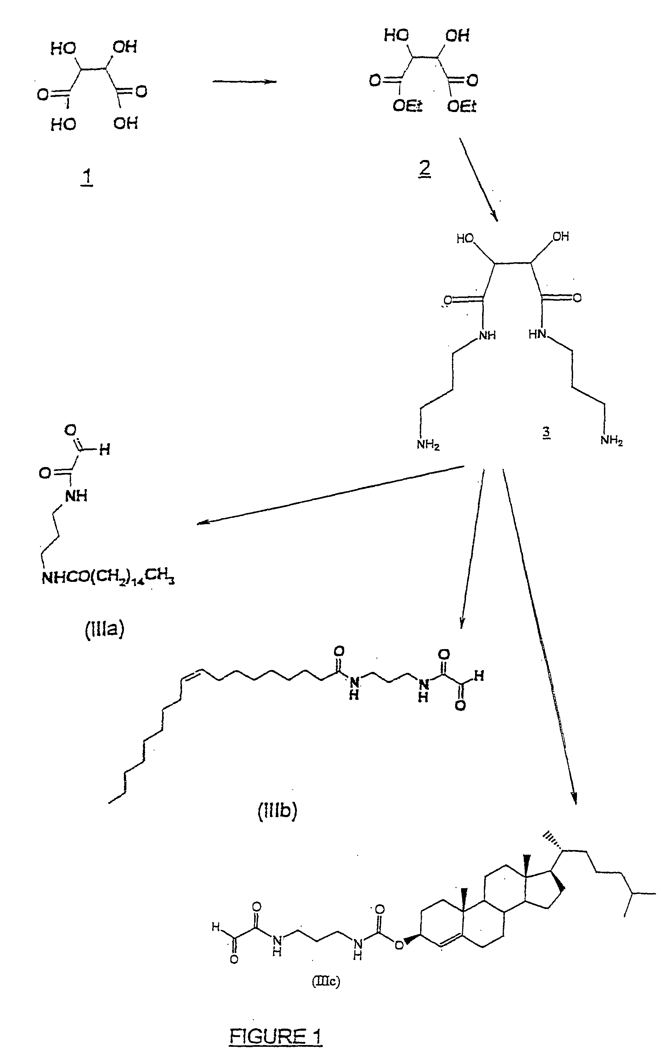

### 1) Synthèse du vecteur lipophile (IIIa) (figure 1).

### • Synthèse du composé 2

Dans un ballon de 500 ml, 10 g (66,6 mmoles) d'acide tartrique 1 sont dissous dans 200 ml d'éthanol absolu, en présence de 1% en masse de résine Amberlyst 15. Le ballon est muni d'un montage soxhlet contenant du tamis moléculaire activé 4 A. L'éthanol est porté à reflux durant 48h. Le milieu réactionnel est ensuite filtré sur verre fritté n°4 pour éliminer la résine. Le filtrat est concentré sous pression réduite et conduit à une huile jaune-clair. Le résidu 2 est séché une nuit sur P₂O₅ et utilisé sans autre forme de purification (m = 13,46 g, rendement = 98,0 %).

L'analyse du composé 2, représenté ci-dessous, par chromatographie sur couche mince donne un indice de migration Rf = 0,76 dans l'éluant CH₂Cl₂/MeOH/H₂O/AcOH (90/10/0,1/0,05).

RMN ¹H (CDCl₃): 1,33 (t, 6H, H_{5,5'}, J₅₋₄=7,2 Hz), 3,28 (s, 2H, H_{1,1'}), 4,33 (q, 4H, H_{4,4'}, J₄₋₅=7,1 Hz), 4,54 (s, 2H, H_{2,2'}). RMN ¹³C : 14,14 (C_{5,5'}), 62,48 (C_{4,4'}), 72,08 (C_{2,2'}), 171,62 (C_{3,3'}).

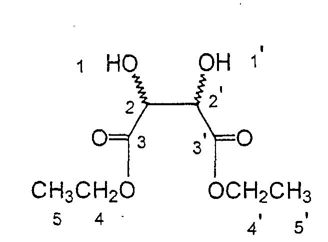

### • Synthèse du composé 3

13,46 mg (65,26 mmoles) du composé 2 sont dissous dans 155 ml d'éthanol absolu et ajoutés goutte à goutte à 56 ml (665,26 mmoles) de 1,3-diaminopropane. Le milieu réactionnel est ensuite agité 3h à température ambiante. L'excès de 1,3-diaminopropane est alors éliminé par évaporation sous pression réduite et entraînement azéotropique en présence d'éthanol (3 x 200 ml). L'huile jaune obtenue est séchée sur P₂O₅ pendant une nuit, puis reprise dans un mélange éthanol/toluène (1/1) et concentrée sous pression réduite (3 × 200 ml). Un composé jaune, sous une forme pâteuse, est ainsi obtenu. Ce composé est précipité dans 50 ml d'un mélange éther éthylique/éthanol (5/1), puis filtré, et le précipité blanchâtre est agité pendant 1h30 à 0°C en présence d'éther éthylique. Il est alors filtré sur fritté et lavé 2 fois avec le minimum d'éther éthylique à froid. Le solide résiduel 3 est séché pendant une nuit sur P₂O₅ (m = 13,92 g, rendement = 81,3%).

L'analyse du composé 3, représenté ci-dessous, est la suivante : Rf = 0,34 dans THF/AcOH/H₂O/AcO⁻NH₄⁺ (35/20/10/1% massique). RMN ¹H (DMSO-d6): 1,48 (q, 4H, H_{6 et 6'}, J_{6-5,6,7 et 6'-5',6',7'}=6,65 Hz), 2,51 (m, 4H, H_{7-7'}), 3,14 et 3,16 (t, 4H, H_{5 et 5'}, J_{5-6 et 5'-6'}=6,3 Hz), 4,19 (s, 2H, H_{2,2'}), 7,75 (t, 2H, H_{4,4'}, J_{4,4'}= 5,97 Hz). RMN ¹³C : 33,51 (C_{6,6'}), 36,98 (C_{7,7'}), 40,06 (C_{5,5'}), 73,43 (C_{2,2'}), 172,87 (C_{5,5'}).

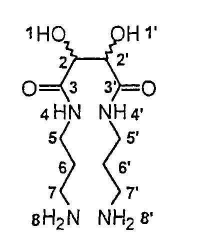

### • Synthèse du composé (IIIa)

996,9 mg (3,8 mmoles) du composé 3 sont dissous dans 15 ml d'eau. Le pH de la solution, égal à 8,5, est ramené à 3,25 à l'aide de 1,46 g d'acide citrique. 1,06 g (4,9 mmoles) de NaIO₄ solide sont alors ajoutés en quatre fois, pendant 5 minutes, au milieu réactionnel maintenu à température ambiante à l'aide d'un bain d'eau. Après 10 minutes d'agitation, 570 mg d'acide tartrique (3,8 mmoles) sont ajoutés pour neutraliser l'excès de NaIO₄ n'ayant pas réagi. Après 10 minutes d'agitation à température ambiante, le pH est ajusté à 8,6 avec 24 ml de N-méthylmorpholine. Le milieu réactionnel est dilué avec 70 ml de 2-méthyl-propan-2-ol et 20 ml d'eau. 2,16 g (1,6 eq.) de palmitoate de succinimidyle sont alors ajoutés à la solution qui est agitée une nuit à température ambiante. Le pH est ensuite ajusté à 3,5 avec 7,11 g d'acide citrique. Le milieu est dilué avec 40 ml d'une solution saturée en NaCl et extrait avec 140 ml de dichlorométhane, puis 100 ml de chloroforme. La phase organique est lavée avec deux fois 100 ml d'une solution saturée en KH₂PO₄, deux fois 100 ml d'une solution saturée en NaCl, puis séchée sur Na₂SO₄, filtrée et concentrée sous pression réduite. Le composé solide résiduel est purifié sur silice en utilisant comme éluant un mélange CH₂Cl₂/AcOEt/EtOH (70/22/5). 951,4 mg du vecteur lipophile (IIIa), soit un rendement de 42,3%, sont ainsi obtenus.

L'analyse du composé (IIIa), représenté ci-dessous sous sa forme d'hydrate, est la suivante : RMN ¹H (DMSO-d6): 0,87 (t, 3H, H₁, J_{1,2}=5,31 Hz), 1,25 (m, 24H, H₂), 1,83 (m, 4H, H₃, H₈), 2,39 (m, 2H, H₄), 3,57 (m, 4H, H₇, H₉), 4,41 (s, 2H, H_{14,15}), 5,50 (m, 1H, H₁₂). RMN ¹³C : 14,31 (C₁), 22,96 (C₂), 26,35 (C₈), 29,99 (C₃), 36,79 (C₄), 37,14 (C₇), 92,18 (C₁₂), 173,42 (C₅), 175,22 (C₁₁). ES-MS : [M+H]⁺ calculé 387,5 ; trouvé 387,2.

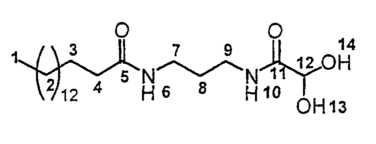

### 2) Synthèse du vecteur lipophile (IIIb) (figure 1).

De l'acide oléique activé sous forme d'ester de N-hydroxysuccinimide est tout d'abord préparé à partir de 859 mg (3,0 mmoles) d'acide oléique, 353 mg (3,0 mmoles) de N-hydroxysuccinimide et 370 µl (2,3 mmoles) de diisopropylcarbo-diimide, qui sont dissous dans 30 ml d'un mélange THF/CH₂Cl₂ (1/1). Après une nuit à 4°C, le milieu est concentré sous pression réduite et utilisé sans autre forme de purification pour la suite de la synthèse.

On procède ensuite de la même façon que pour l'obtention du vecteur lipophile (IIIa), telle que décrite ci-dessus : 500 mg (1,9 mmoles) du composé 3, préparé comme indiqué précédemment, sont dissous dans 10 ml d'eau. Le pH de la solution, égal à 8.5, est ajusté à 3,5 avec 667 mg d'acide citrique. 530 mg (2,4 mmoles) de NaIO₄ solide sont ajoutés en quatre fois, pendant 5 minutes, le milieu étant maintenu à température ambiante à l'aide d'un bain d'eau. Après 10 minutes d'agitation, 286 mg d'acide tartrique (1,9 mmoles) sont ajoutés pour neutraliser l'excès de NaIO₄. Après 10 minutes d'agitation, le pH est ajusté à 8.6 avec 12 ml de N-méthylmorpholine. Le milieu est dilué avec 35 ml de 2-méthyl-propan-2-ol et 20 ml d'eau. L'acide oléique activé sous forme d'ester de N-hydroxysuccinimide est dissous dans 50 ml de 2-méthyl-propan-2-ol et ajouté à la solution. Après une nuit d'agitation, le pH du milieu est ajusté à 3,5 avec 15 g d'acide citrique et dilué avec 20 ml d'une solution saturée en NaCl. Le mélange est extrait avec 70 ml de CH₂Cl₂. La phase organique est lavée deux fois avec 50 ml de solution saturée en KH₂PO₄, puis deux fois avec 50 ml d'une solution saturée en NaCl, séchée sur Na₂SO₄ et concentrée sous pression réduite. Le solide résiduel est purifié sur silice en utilisant comme éluant un mélange CH₂Cl₂/AcOEt/EtOH (70/22/5). 336,3 mg du vecteur lipophile (IIIb), soit un rendement de 27%, sont ainsi obtenus.

### 3) Synthèse du vecteur lipophile (IIIc) (figure 1).

La synthèse est réalisée à partir du *N*-cholestérylcarbonyloxy-succinimide comme décrit pour la préparation du vecteur lipophile (IIIa) ci-dessus. 50,3 mg (0,1905 mmole) du composé 3, préparé comme indiqué précédemment, sont dissous dans 2 ml d'eau. Le pH de la solution égal à 11,05, est ajusté à 3,08 avec 126 mg d'acide citrique. 53,6 mg (0,248 mmole) de NaIO₄ solide sont ajoutés en quatre fois sur 5 minutes, le milieu étant maintenu à température ambiante à l'aide d'un bain d'eau froide. Après 10 minutes d'agitation, 24 mg d'acide tartrique (0,19 mmole) sont ajoutés pour neutraliser l'excès de NaIO₄. Après 10 minutes d'agitation, le pH est ensuite ajusté à 8,51 avec 2,4 ml de *N*-méthylmorpholine. Le milieu est dilué avec 10 ml de 2-méthyl-propan-2-ol. 161,1 mg (0,305 mmole) de Ncholestérylcarbonyloxysuccinimide est ajouté sous forme solide à la solution. Après 1h d'agitation à température ambiante puis 2h à 60°C, 10 ml de CH₂Cl₂ sont ajoutés pour dissoudre totalement le *N*-cholestérylcarbonyloxy-succinimide. Après 1 heure d'agitation à température ambiante, le pH de la phase aqueuse est ajusté à 3,8 avec 1,8 g l'acide citrique. Le mélange est extrait avec 15 ml de CH₂Cl₂. La phase organique est lavée deux fois avec 30 ml de solution saturée en KH₂PO₄ puis avec 30 ml d'une solution saturée en NaCl (avec ajout de 15 ml d'éther éthylique pour permettre la séparation des phases). La phase organique est ensuite séchée sur Na₂SO₄ et concentrée sous pression réduite. Le solide résiduel est purifié sur gel de silice en utilisant comme éluant un mélange CHCl₃/AcOH (95/5) pour fournir 53,4 mg du vecteur lipophile (IIIc), soit un rendement de 32,2 %.

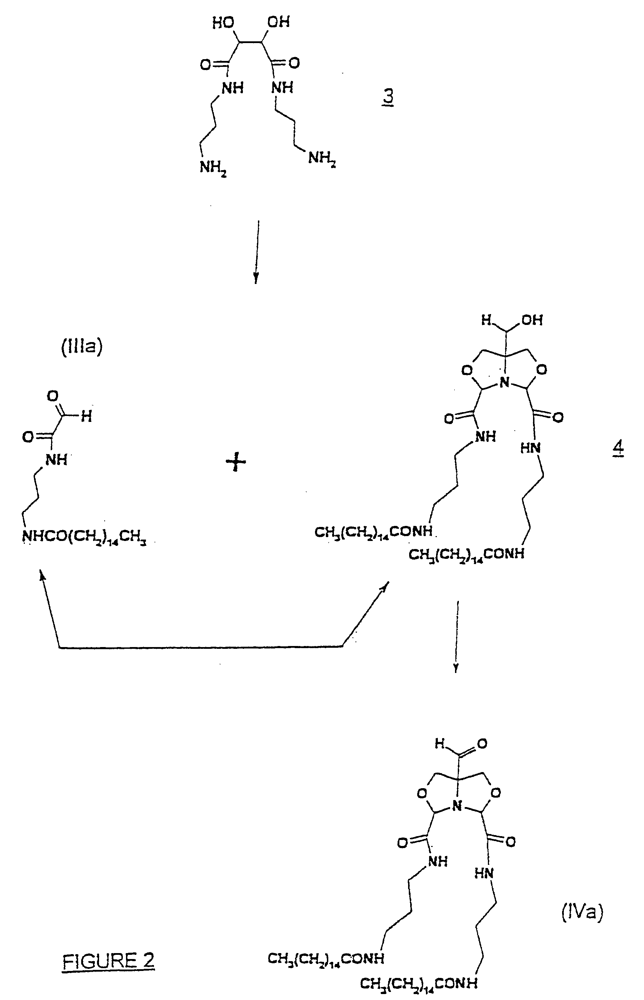

### 4) Synthèse du vecteur lipophile (IVa) (figure 2).

1 g (3,8 mmoles) du composé 3, dont la synthèse a été décrite précédemment, est dissous dans 10 ml d'eau. Le pH est ajusté à 3,25 avec de l'acide citrique (1,8 g). 1,06 g (4,95 mmoles) de NaIO₄ sont alors ajoutés en 4 fois au milieu réactionnel maintenu à température ambiante à l'aide d'un bain d'eau. Une fois l'addition terminée, le mélange est agité pendant 10 minutes. L'avancement de la réaction est suivi par chromatographie sur couche mince en utilisant l'éluant THF/AcOH/H₂O/AcO⁻NH₄⁺ (35/20/10/1% massique).

L'excès de NaIO₄ est consommé par ajout de 3 eq. d'acide tartrique. Après 5 minutes d'agitation à température ambiante, le pH du milieu réactionnel est ajusté à 8,55 par ajout de 7 g de tris(hydroxyméthyl)aminométhane. 70 ml de tert-butanol sont ajoutés, ainsi que 1,21 g (3,4 mmoles) de palmitoate de succinimidyle solide. Le mélange est agité fortement durant 1 nuit, dilué avec 50 ml d'eau saturée en KH₂PO₄, puis extrait deux fois avec un mélange CH₂Cl₂/tert-butanol (100 ml/50 ml). La phase organique est lavée deux fois avec une solution saturée en NaCl puis séchée sur MgSO₄ et concentrée sous pression réduite. Le solide jaune pâteux résiduel est dissous dans de l'éthanol et la solution est concentrée sous pression réduite. L'opération est réalisée deux fois afin d'éliminer le tert-butanol résiduel. On obtient le composé 4, qui correspond au vecteur lipophile (IVa) sous forme réduite, sous la forme d'un solide blanc, en mélange avec le vecteur lipophile (IIIa).

La purification de 301,4 mg de ce solide par chromatographie sur silice avec un mélange CH₂Cl₂/AcOEt/EtOH (70/20/10) permet d'obtenir 157,7 mg (rendement = 52,2%) du composé 4 et 37,6 mg (rendement = 13,2%) du vecteur lipophile (IIIa).

L'analyse du composé 4, représenté ci-dessous, est la suivante : RMN ¹H (pyridine-d5): 0,86 (t, 6H, H_{1,1'}, J=6,30 Hz), 1,25 (m, 48H, H_{2,2'}), 1,83 (m, 8H, H_{3,3',8,8'}), 2,37 (t, 2H, H₄, J=7,20 Hz), 2,40 (t, 2H, H_{4'}, J=7,20 Hz), 3,60 (m, 8H, C_{7,7',9,9'}), 3,99 (s, 2H, H₁₅), 4,07 (s, 2H, H₁₃), 4,08 (d, 1H, H_{13'}, J_{13'-13''}=8,70 Hz), 4,48 (d, 1H, H_{13''}, J_{13''-13'}=8,70 Hz), 5,40 (s, 1H, H₁₂), 5,50 (s, 1H, H_{12'}), 6,80 (t, 1H, H₁₆, J=5,10 Hz), 8,50 et 8,55 (t, 2H, H_{6,6'}, J_{6-7 ou 6'-7'}=5,72 Hz), 8,89 et 9,07 (t, 2H, H_{10,10'}, J_{10-9 ou 10'-9'}=6,16 Hz). RMN ¹³C : 15,60 (C_{1,1'}), 24,25 (C_{2,2'}), 27,58 (C_{3,3'}), 31,15 (C_{8,8'}), 37,54 (C_{4,4'}), 38,02 (C_{7,7',9,9'}), 65,29 (C₁₅), 74,00 (C₁₃), 75,5 (C_{13'}), 76,18 (C_{13''}), 93,70 et 94,90 (C₁₂ ou C_{12'}), 171,45 (C_{5,5'}), 175, 29 (C_{11,11'}). ES-MS : [M+H]⁺ calculé 823,2 ; trouvé 822,7.

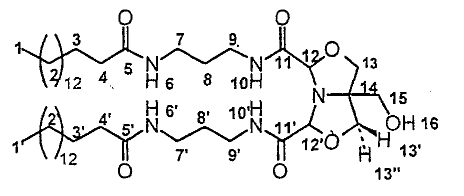

Le composé 4 est ensuite oxydé afin d'obtenir le vecteur lipophile (IVa) : 40 mg (48,6 µmoles) du composé 4 sont dissous dans 750 µl de CH₂Cl₂ et ajoutés à 61,8 mg (145,8 µmoles) de Dess-Martin periodinane (c'est-à-dire le composé 1,1,1-triacétoxy-1,1-dihydro-1,2-benziodexol-3(1H)-one, tel que décrit dans J. Org. Chem., 1983, 48, 4156 et dans J. Am. Chem. Soc., 1991, 113, 7277) dissous dans 1,5 ml de CH₂Cl₂. Le mélange est agité 18h à température ambiante. Le milieu réactionnel est ensuite dilué avec 10 ml de CH₂Cl₂, 10 ml d'éther éthylique et 10 ml d'une solution saturée de NaHCO₃. 46,1 mg de Na₂S₂O₄ sont ensuite ajoutés à la solution. Après 5 minutes d'agitation, 5 ml d'éther éthylique et 5 ml de CH₂Cl₂ sont ajoutés et les deux phases sont séparées. La phase organique est lavée deux fois avec 15 ml d'une solution saturée en NaHCO₃, séchée sur MgSO₄, filtrée et concentrée sous pression réduite. L'huile jaune résiduelle est purifiée par chromatographie sur silice en utilisant un mélange CH₂Cl₂/AcOEt/EtOH/Et₃N (70/20/15/0,5%). 23 mg (57,7 %) du vecteur lipophile (IVa) sont ainsi obtenus.

L'analyse du vecteur lipophile (IVa), représenté ci-dessous, est la suivante : RMN ¹H (pyridine-d5): 0,87 (t, 6H, H_{1,1'}, J=6,30 Hz), 1,26 (m, 48H, H_{2,2'}), 1,83 (m, 8H, H_{3,3',8,8'}), 2,39 (m, 4H, H_{4,4'}), 3,60 (m, 8H, C_{7,7',9,9'}), 4,21 (m, 4H, H_{13,13'}), 5,60 (m, 2H, H_{12,12'}), 8,46 (m, 2H, H_{6,6'}), 9,02 (m, 2H, H_{10,10'}). RMN ¹³C : 14,30 (C_{1,1'}), 22,95 (C_{2,2'}), 26,32 (C_{3,3'}), 29,86 (C_{8,8'}), 36,75 (C_{4,4'}), 36,75 (C_{7,7',9,9'}), 73,5 (C_{13,13'}), 93,5 (C_{12,12'}), 166,9 (C_{5,5'}), 170,7 (C_{11,11'}). ES-MS : [M+H]⁺ calculé 821,2 ; trouvé 820,6.

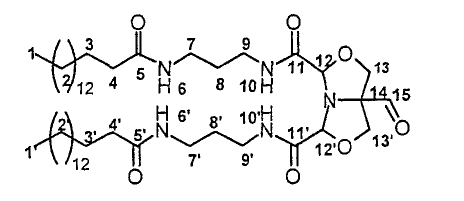

### EXEMPLE 5 : Autre protocole de synthèse du vecteur lipophile (IIIa) (figure 2).

En variante des protocoles décrits dans l'exemple 2, le vecteur lipophile (IIIa) conforme à l'invention peut être synthétisé à partir du vecteur lipophile (IVa) sous forme réduite (c'est-à-dire le composé 4), selon le protocole suivant.

245,0 mg du mélange du vecteur lipophile (IIIa) avec le composé 4, obtenu dans l'exemple 2 avant purification, sont dissous dans un mélange tert-butanol/H₂O/TFA 10% dans l'eau (15 ml/10 ml/1 ml). Le mélange est agité une nuit à température ambiante, puis dilué avec une solution saturée en NaCl (15 ml). Le milieu réactionnel est ensuite extrait deux fois avec un mélange CH₂Cl₂/tert-butanol (20 ml/20 ml). La phase organique est lavée 2 fois avec 15 ml d'une solution saturée en NaCl, filtrée et concentrée sous pression réduite. Le solide blanc (IIIa) ainsi obtenu est purifié par chromatographie sur silice dans les conditions décrites dans l'exemple 2 en rapport avec l'obtention du vecteur lipophile de formule (IIIa). On obtient 136,2 mg du composé (IIIa), soit un rendement de 56,2%.

### EXEMPLE 6 : Autre protocole de synthèse du vecteur lipophile (IVa) (figure 2).

En variante du protocole décrit dans l'exemple 2, il est possible d'obtenir le composé 4, c'est-à-dire le vecteur lipophile (IVa) sous forme réduite, à partir du vecteur lipophile (IIIa), selon le protocole suivant :
une solution à 242,3 mg/ml de tris(hydroxyméthyl)-aminométhane dans l'eau est préparée puis ajustée à pH 8,5 avec de l'acide chlorhydrique concentré. Cette solution est ensuite saturée en NaCl, puis filtrée. 20,25 mg (55,0 µmoles) de vecteur lipophile (IIIa) dissous dans 600 µl de 2-méthyl-propan-2-ol sont ajoutés à 600 µl de la solution précédemment préparée. Le milieu réactionnel est agité à température ambiante pendant 4 heures, dilué avec 2 ml d'eau et le produit est extrait avec 2 ml d'une solution CH₂Cl₂/2-méthyl-propan-2-ol (1/1). La phase organique est lavée deux fois avec 1 ml d'une solution saturée en KH₂PO₄, puis séchée sur Na₂SO₄, filtrée et concentrée sous pression réduite. 20,14 mg (54,7 µmoles) de vecteur lipophile (IIIa) dissous dans 2 ml de 2-méthyl-propan-2-ol sont ajoutés au résidu solide obtenu précédemment. Après 13 h d'agitation à 30°C, le milieu réactionnel est dilué avec 4 ml de CH₂Cl₂. La phase organique est lavée avec 2 ml d'une solution saturée en NaCl, séchée sur Na₂SO₄, filtré et concentrée sous pression réduite. Le résidu solide est purifié sur colonne de silice en utilisant comme éluant le mélange CH₂Cl₂/AcOEt/EtOH (70/22/12) pour conduire à 23,1 mg de composé 4, soit un rendement de 51%). Ce dernier est ensuite oxydé, comme décrit dans l'exemple 2, pour conduire au vecteur lipophile (IVa).

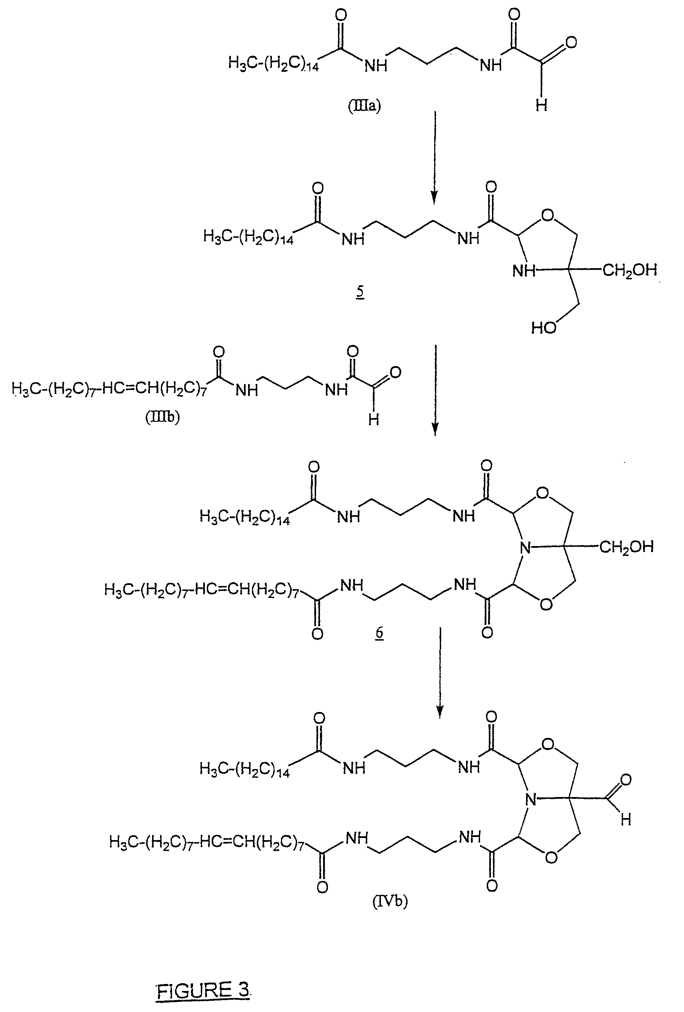

### EXEMPLE 7 : Synthèse du vecteur lipophile (IVb) (figure 3)

En variante du protocole de synthèse du vecteur lipophile (IVa), il est possible d'obtenir le composé 6, c'est-à-dire le vecteur lipophile (IVb) sous forme réduite, à partir des vecteurs lipophiles (IIIa) et (IIIb), selon le protocole suivant :
une solution à 242,3 mg/ml (2 mol/l) de tris(hydroxyméthyl)-aminométhane dans l'eau est préparée puis ajusté à pH 8,5 avec de l'acide chlorhydrique concentré. Cette solution est ensuite saturée en NaCl puis filtrée. 99,9 mg (270 mmole) du vecteur lipophile (IIIa) dissous dans 3 ml de 2-méthyl-propan-2-ol sont ajoutés à 3 ml de la solution préparée précédemment. Le milieu réactionnel est agité à température ambiante pendant 6 heures, dilué avec 5 ml d'eau et le produit est extrait avec 5 ml d'une solution de CH₂Cl₂/2-méthyl-propan-2-ol (1/1). La phase organique est lavée deux fois avec 2 ml d'une solution saturée de KH₂PO₄, puis séchée sur Na₂SO₄, filtrée et concentrée sous pression réduite pour conduire à l'oxazolidine palmitoylée 5, sous la forme d'un résidu solide.

99,2 mg (270 mmole) de vecteur lipophile (IIIb) dissous dans 10 ml de 2-méthyl-propan-2-ol sont ensuite ajoutés à l'intermédiaire 5, obtenu précédemment. Après 20 heures d'agitation à 30°C, le milieu réactionnel est dilué avec 20 ml de CH₂Cl₂. La phase organique est lavée avec 10 ml de solution saturée en NaCl, séchée sur Na₂SO₄, filtrée et concentrée sous pression réduite. Le résidu solide est purifié sur gel de silice en utilisant le mélange CH₂Cl₂/AcOEt/EtOH (70/22/12) pour obtenir 133 mg de composé 6, soit un rendement de 53 %.

Caractérisation de l'oxazolidine 5 : ¹H RMN (300 MHz, CDCl₃) : δ (ppm) : 0,75 (t, 3 H, *J*₁₋₂ = 6,7 Hz, H1), 1,25 (m, 24 H, H2), 1,80 (m, 4 H, H3 et H8), 2,33 (t, 2 H, *J*₃₋₄ = 7,49 Hz, H4), 3,53 (m, 4 H, H7 et H9), 4,04 (s, 2 H, H13), 4,20 (s, 4 H, H15 et H15'), 4,23 (s, 1 H, H17), 5,41 (s, 1 H, H12), 8,39 (t, 1 H, J₆₋₇ = 5,7 Hz, H6), 8,99 (t, 1 H, J₁₀₋₉ = 5,9 Hz, H10) ; ¹³C (300 MHz, CDCl₃) : δ (ppm) : 14,8 (C1), 26,8 (C3), 30,9 (C8), 23,4, 30,3 et 37,6 (C2), 37,2 (C4,7,9), 63,9 et 70,8 (C15 et C15'), 64,4 (C13), 68,8 (C14), 90,9 (C12), 171,4 (C5), 174,1 (C11) ; MALDI-TOF-MS : [M+H+] calculé 471,7; trouvé 471,2.

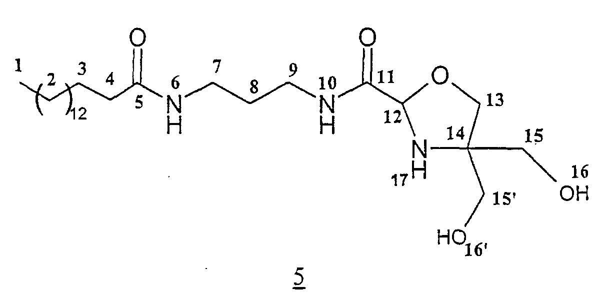

Caractérisation de la bis-oxazolidine 6 : RMN ¹H : δ (ppm) 0,84 (m, 6 H, H1 et H1') ; 1,23 (m, 42 H, H2 et H2') ; 1,78 (m, 8 H, H3, H3',H8 et H8') ; 2,05 (m, 4 H, H17 et H17') ; 2,34 (m, 4 H, H4 et H4') ; 3,50 (m, 8 H, H7, H7',H9 et H9') ; 3,95 (s, 2 H, H15) ; 4,03 (s, 2 H, H13) ; 4,05 (d, 1 H, H13', *J*_{13'-13''} = 8,9 Hz) ; 4,45 (d, 1 H, H_{13''}, *J*_{13''-13'} = 8,8 Hz) ; 5,36 (m, 2 H, H18 et H18') ; 5,37 et 5,45 (s, 2 H, H12 et H12') ; 8,35 et 8,45 (t, 2 H, *J*₆₋₇ = 5,7 et *J*_{6-7'} = 5,9 Hz H6 et H6') ; 8,78 et 9,0 (t, 2 H, *J*₁₀₋₉ = 6,1 et *J*_{10'-9'} = 6,0 Hz, H10 et H10') ; RMN C¹³ : δ (ppm) 14,4 (C1 et C1') ; 23,1 , 29,8 et 32,3 (C2 et C2') ; 29,8 (C3 et C3') ; 26,4 (C8 et C8') ; 27,6 (C17 et C17') ; 36,8 (C4 et C4') ; 36,4 et 36,7 (C7 , C7', C9 et C9') ; 64,1 (C15 et C15') ; 72,6 (C13) ; 74,3 et 75,0 (C13' et C13") ; 92,6 et 93,8 (C12 et C1.2') ; 130,3 (C18 et C18') ; 166,9 , 170,3 , 173,8 et 174,1 (C5, C5', C10, C10'). MALDI-TOF-MS: [M+H⁺] : calculé 849,3 ; trouvé 848,6. 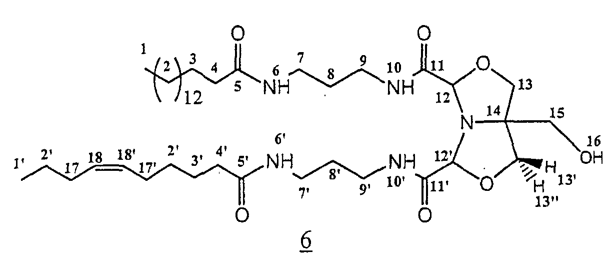 Ce dernier est ensuite oxydé, comme décrit dans l'exemple 2, pour conduire au vecteur lipophile (IVb) : MALDI-TOF-MS : [M+H⁺] calculé 847,2 ; trouvé 846,6.

### EXEMPLE 8 : Couplages, en solution, entre un ou plusieurs peptides et un vecteur lipophile de nature non peptidique portant une fonction aldéhyde.

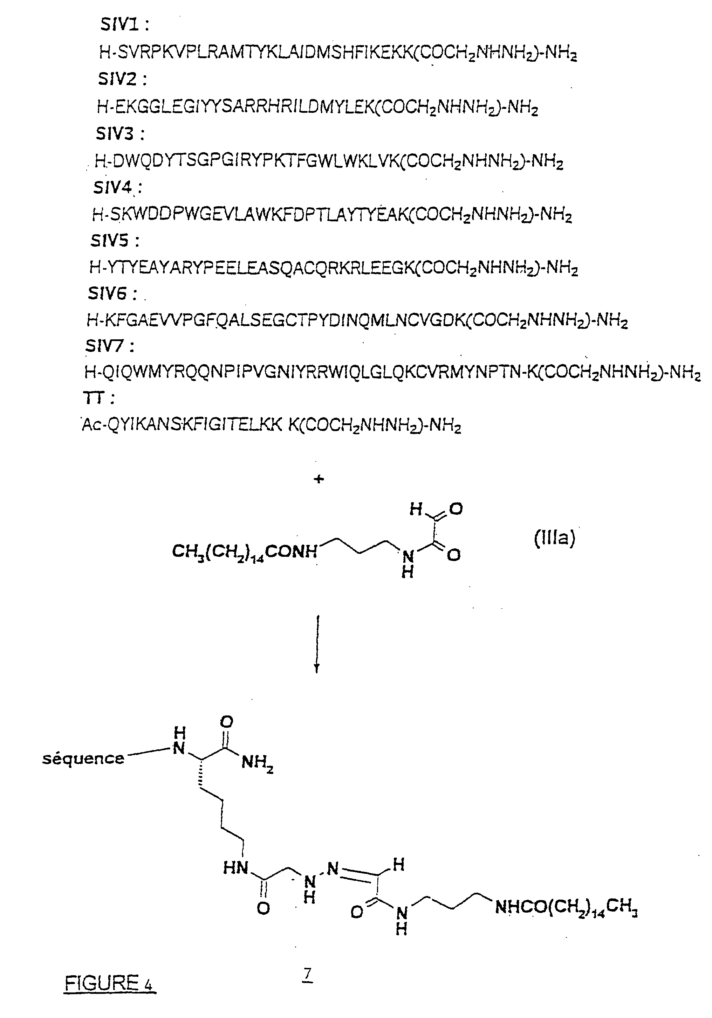

### 1) Couplage du mélange des peptides SIV1-7 et TT avec le vecteur lipophile (IIIa) (figure 4).

Le mélange des hydrazinopeptides SIV1-7 et TT, préparés conformément à l'exemple 1, est réalisé à partir d'environ 2 mg de chaque lyophilisat. Une solution du vecteur lipophile (IIIa), préparé conformément à l'exemple 2 ou à l'exemple 3, en quantité de 1,1 équivalents par rapport au total des fonctions hydrazinoacétiques portées par les peptides (en tenant compte des contre-ions et de la teneur en peptide de chaque lyophilisat) dans le tert-butanol est préparée. La réaction de couplage (ligation chimique) a lieu dans un mélange tert-butanol/eau (95/5 en volume), la concentration finale en peptide étant de l'ordre de 1,3 mmol/l. L'eau desionisée est d'abord ajoutée sur le mélange d'hydrazinopeptides de façon à mouiller ceux-ci, la solution du vecteur lipophile (IIIa) dans le tert-butanol étant ensuite aj outée.

La réaction de ligation, aboutit à l'obtention du mélange de lipopeptides 7, le terme « séquence » (figure 4) désignant la séquence peptidique des peptides SIV1-7 ou TT. Cette réaction est suivie par RP-HPLC comme suit. 20 µl du milieu réactionnel sont prélevés, sur lesquels sont ajoutés 80 µl d'un mélange acide acétique/eau (20/80). 50 µl de ce volume sont injectés sur une colonne C3 analytique (Zorbax , diamètre de 4.6 mm, longueur de 300 mm) avec un gradient en solvant B (tel que celui décrit dans l'exemple 1 en rapport avec la purification des peptides SIV1-7 et TT) de 0 à 100% en 30 minutes (1 ml/min, 215 nm, 50°C). Cette technique permet de suivre la conversion des hydrazinopeptides en peptides ligués sur le même chromatogramme, les hydrazinopeptides et les peptides liés chimiquement au vecteur lipophile (peptides « ligués ») ne présentant pas les mêmes temps d'élution. Le rapport de la somme des intégrations des pics des hydrazinopeptides et des peptides ligués permet d'estimer le taux de conversion de la réaction.

Après environ 20 heures de réaction de couplage, des fractions du milieu réactionnel sont lyophilisées et on procède à une analyse LC-MS des lipopeptides en mélange. A cette fin, le système HPLC Hewlett-Parckard est connecté, par un capillaire, à un spectromètre de masse Micromass Quatro. Les conditions de l'HPLC sont les mêmes que celles utilisées pour le suivi de la réaction de couplage, le débit utilisé étant toutefois de 0.2 ml/min sur une colonne C3 (Zorbax, diamètre de 2.1 mm, longueur de 200 mm). Le mélange des hydrazinopeptides seul (solution aqueuse à 1 mg/ml) est injecté à raison de 20 µl avec un gradient de 0 à 100% en solvant B, pendant 30 minutes. L'ordre de sortie des hydrazinopeptides, leurs temps de rétention (rt, en minutes) et leurs poids moléculaires observés sont les suivants : TT (17.1 ; 2222) ; SIV5 (17.5 ; 3452) SIV2 (18.3 ; 2968) ; SIV1 (19.4; 3258) ; SIV6 (20.9 ; 3501.5) ; SIV7 (21.0 ; 4804) ; SIV4 (21.2 ; 3186) ; SIV3 (21.6 ; 3112).

La réaction de couplage est arrêtée après environ 20 heures et le milieu réactionnel, lyophilisé, est remis en solution dans de l'eau à raison de 1 mg/ml. Cette solution est injectée selon les mêmes conditions que décrites ci-dessus pour le mélange de peptides, excepté un gradient plus résolutif allant de 0 à 50% en solvant B pendant 10 minutes, puis de 50 à 100% pendant 30 minutes, suivi de quelques minutes à 100%. A ce stade, il est toujours possible de détecter en HPLC et en LC-MS la présence des hydrazinopeptides SIV1-7 et TT. Il sont cependant masqués en partie par le bruit de fond et les impuretés s'accumulant au cours de la première partie du gradient en solvant B. Cinq pics majoritaires sont observés en HPLC, avec en parallèle six pics TIC (Total Ion Count) en LC-MS. Ces pics correspondent aux hydrazinopeptides SIV1-7 et TT couplés au vecteur lipophile (IIIa), dont certains co-éluent. Il est cependant possible de déterminer leur ordre de sortie et leurs masses observées, comme indiqué dans le tableau II.

**Tableau II**

| | [M+H]⁺ calculé | [M+H]⁺ observé | rt (minutes) (LC/MS) |
|---|---|---|---|
| SIV1 | 3608,89 | 3608 | 19,4 |
| SIV5 | 3803,716 | 3802 | 19,6 |
| TT | 2572,57 | 2572 | 19,9 |
| SIV2 | 3319,35 | 3318 | 20,4 |
| SIV7 | 5156,5 | 5155 | 21,2 |
| SIV4 | 3538,465 | 3538 | 23,1 |
| SIV6 | 3852,9 | 3851,5 | 23,1 |
| SIV3 | 3463,49 | 3462 | 24,5 |

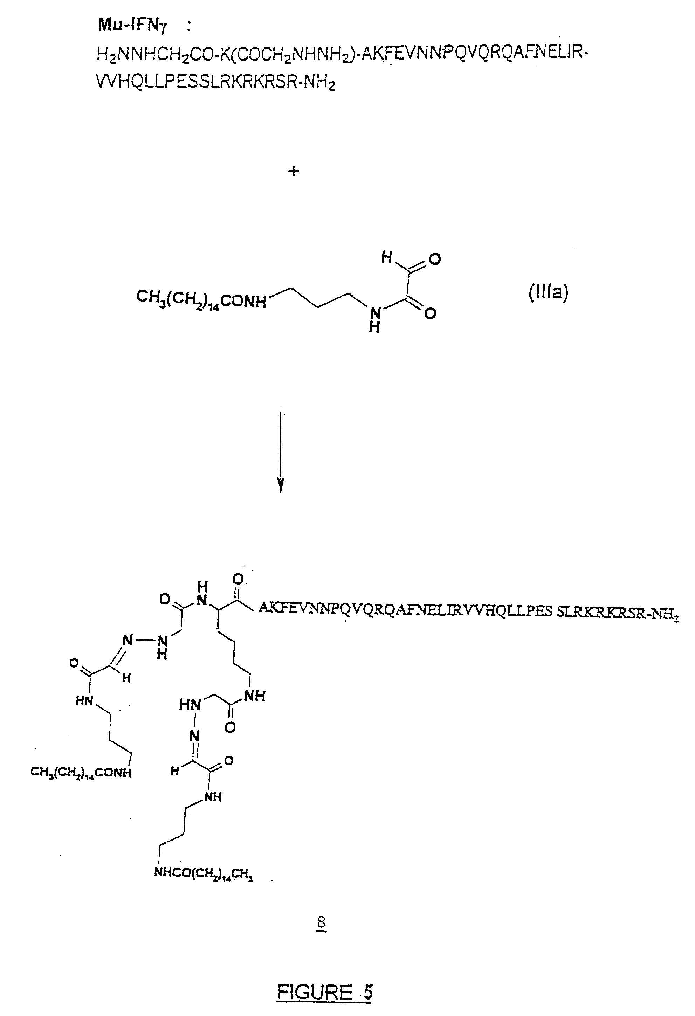

### 2) Couplage du peptide Mu-IFNγ avec deux vecteurs lipophiles (IIIa) (figure 5).

708,2 µg (1,92 µmoles) du vecteur lipophile (IIIa), préparé conformément à l'exemple 2 ou à l'exemple 3, dissous dans 640 µl de tert-butanol, sont ajoutés à 5,04 mg (soit environ 1 µmole) du peptide Mu-IFNγ, préparé conformément à l'exemple 1, préalablement mis en suspension dans 35 µl d'eau. Après 5h d'agitation à température ambiante, le milieu réactionnel est congelé et lyophilisé. L'analyse par ES-MS du lipopeptide 8 résultant est la suivante : [M+H]⁺ calculé 5547,7, trouvé 5547,5.

### 3) Couplage des peptides Mu-IFNγ a et Mu-IFNγ b avec le vecteur lipophile (IIIa).

1,067 mg de vecteur lipophile (IIIa) dissous dans 1,88 ml de tert-butanol sont ajoutés à 10 mg de peptide (à savoir soit le peptide Mu-IFNγ a, soit le peptide Mu-IFNγ b) dans 105 µl d'eau. Après 4 h à température ambiante, la solution est diluée avec le même volume d'eau et lyophilisée. On obtient 9,2 mg de lipopeptide à partir de Mu-IFNγ a (lipopeptide dénommé « L-Mu-IFNγ a ») et 9,3 mg de lipopeptide à partir de Mu-IFNγ b (lipopeptide dénommé « L-Mu-IFNγ b »). Leur analyse par ES-MS est la suivante : L-Mu-IFNγ a : [M+H]⁺ calculé 3008,7 ; trouvé 3007,5. L-Mu-IFNγ b : [M+H]⁺ calculé 3008,7 ; trouvé 3007,0.

### 4) Couplage des peptides Mu-IFNγ c, Mu-IFNγ d, Mu-IFNγ a et Mu-IFNγ b avec le vecteur lipophile (IIIc).

5,8 mg de vecteur lipohile (IIIc) sont dissous dans 6,302 ml de 2-méthyl-propan-2-ol.

A 5 mg (1,32 µmol) de peptide (à savoir soit le peptide Mu-IFNγ a, soit le peptide Mu-IFNγ b) dissous dans 50 µl d'eau sont ajoutés 965 µl (1,58 µmol) de la solution contenant le vecteur lipophile (IIIc) préparée ci-dessus. Après 5 heures de réaction à température ambiante, la solution est diluée par le même volume d'eau et lyophilisée. On obtient 6,3 mg de lipopeptide à partir de Mu-IFNγ a (lipopeptide nommé Cho-Mu-IFNγ a) et 6,4 mg de lipopeptide à partir de Mu-IFNγ b (lipopeptide nommé Cho-Mu-IFNγ b). Leur analyse par MALDI-TOF-MS est la suivante :
Cho-Mu-IFNγ a : *m*/*z*(M+H)⁺ calculé 3184,0 ; trouvé 3183,2.
Cho-Mu-IFNγ b : *m*/*z*(M+H)⁺ calculé 3184,0 ; trouvé 3184,3.

De la même manière, à 8 mg (1,30 µmol) de peptide (à savoir soit le peptide Mu-IFNγ c, soit le peptide Mu-IFNγ d) dissous dans 50 µl d'eau sont ajoutés 952 µl de la solution contenant le vecteur lipophile (IIIc). Après 5h de réaction à température ambiante, la solution est diluée avec le même volume d'eau et lyophilisée. On obtient 8,6 mg de lipopeptide à partir de Mu-IFNγ c [lipopeptide nommé Cho-Mu-IFNγ c ou 9 figure 7)] et 8,4 mg de lipopeptide à partir de Mu-IFNγ d (lipopeptide nommé Cho-Mu-IFNγ d). Leur analyse par MALDI-TOF-MS est la suivante :
Cho-Mu-IFNγ c : *m*/*z*(M+H)⁺ calculé 5298,4 ; trouvé 5298,0.
Cho-Mu-IFNγ d : *m*/*z*(M+H)⁺ calculé 5298,4 ; trouvé 5299,7.

### 5) Couplage du peptide G18R avec le vecteur lipophile (IIIb).

15 mg (6,75 µmoles) de peptide sont imbibés avec 254 µl d'eau, puis 3,73 mg de vecteur lipophile (IIIa) en solution dans 4,82ml de tert-butanol sont ajoutés. Après 48 h à température ambiante, la solution est lyophilisée. On obtient 17 mg de lipopeptide 6, dont l'analyse par TOF-PDMS est la suivante : [M+H]⁺ calculé 2259,8, trouvé 2259,4.

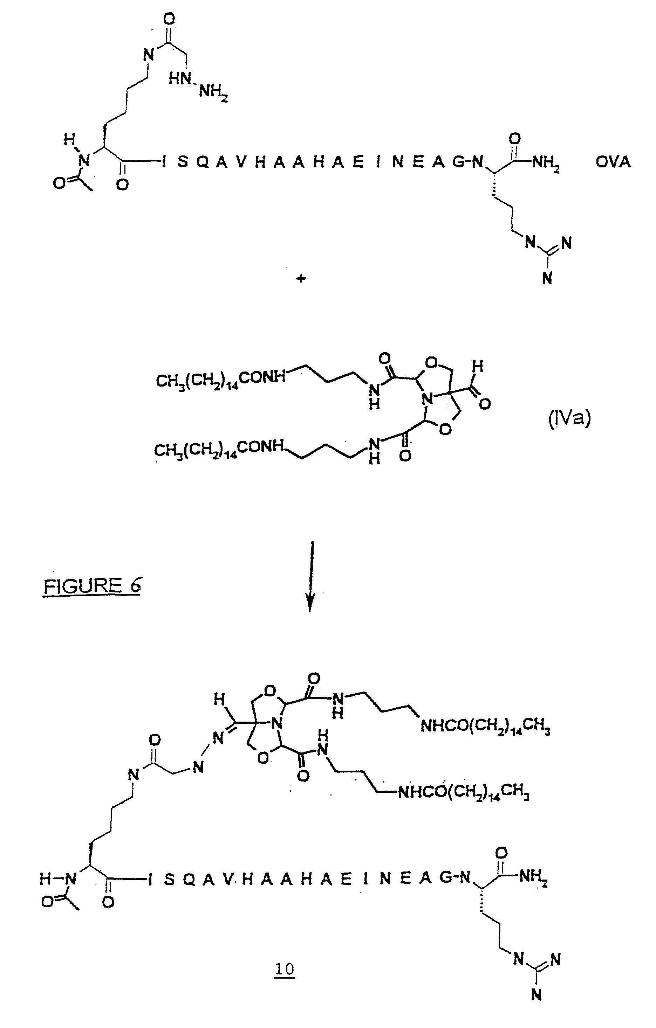

### 6) Couplage du peptide OVA avec le vecteur lipophile (IVa) (figure 6).

1 mg (0,40 µmoles) de peptide OVA, préparé conformément à l'exemple 1, est dissous dans 75 µl d'un tampon phosphate/citrate 25 mM, de pH 6.2. Ce tampon est obtenu à l'aide de 160.5 µl d'une solution de Na₂HPO₄ 0.2M et 89,5 µl d'acide citrique 0.1M, le mélange étant complété à 1 ml avec de l'eau. 663 µg (0,81 µmoles) du vecteur lipophile (IVa), préparé conformément à l'exemple 2 ou à l'exemple 6, sont dissous dans 75 µl de tert-butanol. Les deux solutions sont ensuite mélangées et chauffées à 50°C durant 5 h. L'avancement de la réaction est suivi par HPLC sur une colonne C3 Zorbax en utilisant un gradient linéaire de 0 à 100% de solvant B constitué d'un mélange 80% acétonitrile / 20% TEAP 25 mM, pH 7,1 pendant 30 minutes, puis 10 minutes à 100% en solvant B (débit de 1 ml/min, détection à 215 nm). Après 5h, le lipopeptide 10 est précipité par ajout de 500 µl d'eau, centrifugé et repris dans 500 µl d'isopropanol. Le lipopeptide brut est analysé par ES-MS : [M+H]⁺ calculé 2818,4 ; trouvé 2818,6.

### 7) Couplage du glycomimétique) [(Qui)₄AKHdz] avec le vecteur lipophile (IIIa).

9,4 mg (6,4 mmol) du composé (Qui)₄AKHdz sont dissous dans 244 mL d'eau puis ajoutés à 2,44 mg du vecteur lipophile (IIIa) en solution dans 5,07 ml de 2-méthyl-propan-2-ol. Le milieu réactionnel est agité pendant 5 heures à 30°C puis dilué par de l'eau, congelé et lyophilisé pour fournir quantitativement le dérivé QuiAK-Pam sous la forme d'un résidu blanc.

MALDI-TOF : *m*/*z* (M+Na)⁺ calculé 1742,0 ; trouvé 1742,9 ; (M+K)⁺ calculé 1758,1 ; trouvé 1757,9. (Qui)₄AK-Pam.

### 8) Couplage du mélange des peptides SIV1-7, TT et (Qui)₄AKHdz avec le vecteur lipophile (IIIa).

Le protocole suivi est voisin de celui décrit EXEMPLE 8, 1).

52,5 mg d'un mélange équi-massique de chacun des peptides SIV1-7 (correspondant à une valeur moyenne de 12,1 mmoles de chacun des peptides, en tenant compte des contre ions et de la teneur en peptide de chaque lyophilisat) sont dissous dans 0,464 mL d'eau. Parallèlement, 12,94 mg (8,72 mmoles) du composé (Qui)₄AKHdz sont dissous dans 0,335 mL d'eau. Aux deux solutions, préalablement réunies, sont ajoutés 9,63 mg (24,9 mmoles, 1,2 équivalent) de vecteur lipophile (IIIa) en solution dans 15,198 ml de 2-méthyl-propan-2-ol. La concentration finale de chacun des peptides est donc d'environ 1,3 mmol.l⁻¹ dans un mélange 2-méthyl-propan-2-ol/H₂O (95/5). Le milieu réactionnel devient limpide instantanément. Ce dernier est agité sous azote à 30°C. Le suivi par RP-HPLC [pratiqué comme décrit 1)] de l'avancement de la réaction nous indique que les ligations sont presque complètes après seulement 5 minutes. Après 5h, le milieu réactionnel est dilué à l'eau, congelé puis lyophilisé. Une RP-HPLC micro-préparative est pratiquée sur un échantillon du brut réactionnel pour permettre l'identification de chacun des lipopeptides par analyse de spectrométrie de masse.

| | [M+H]⁺ calculé | [M+H]⁺ trouvé | Fragment peptide parent |
|---|---|---|---|
| SIV1 | 3623,9 | 3625,4 | 3276,0 |
| SIV5 | 3803,7 | 3803,2 | 3453,9 |
| SIV2 | 3319,3 | 3319,0 | 2969,7 |
| SIV7 | 5156,5 | ? | ? |
| SIV4 | 3538,5 | ? | 3188,8 |
| SIV6 | 3852,9 | ? | 3503,9 |
| SIV3 | 3463,5 | 3464,2 | 3113,9 |
| QuiAK-Pam | 1742,0^{a} | 1742,0^{a} | |

| | | | |
|---|---|---|---|
| a : sous forme d'adduit sodium | | | |

### 9) Couplage entre les composés H₂N-KVGFFKR-NH₂ et H₂N-VKYAL-NH₂ et le vecteur lipophile (IIIa).

1,73 mg (4,5 mmoles) et 0,68 mg (1,8 mmoles) du vecteur lipophile (IIIa) sont dissous dans 3,60 et 1.41 ml de 2-méthyl-propan-2-ol, respectivement, puis ajoutés à 5,8 mg (4,3 mmoles) du composé H₂N-KVGFFKR-NH₂ dissous dans 190 ml d'H₂O et à 1,4 mg (1,7 mmole) du composé H₂N-VKYAL-NH₂ dissous dans 77 ml d'H₂O, respectivement. Les milieux réactionnels sont agités pendant 5 heures à 30 °C puis dilués par de l'eau, congelés et lyophilisés pour fournir les composés 11 et 12, respectivement.

4,5 mg (66 mg) du composé 13 sont obtenus après purification RP-HPLC semi-préparative sur un système Shimadzu LC-4A, en utilisant une colonne C3 Zorbax, à un débit de 3 ml min⁻¹, à 60°C et une détection à 215 nm : solvant système A : 0,05% TFA dans de l'eau désionisée ; solvant système B : 0,05% TFA dans de l'isopropanol à 60% dans de l'eau désionisée (gradient: de 20 à 40% de tampon B en 10 minutes puis isocratique 10 minutes puis de 40% à 100% en 10 minutes et isocratique pendant 20 minutes) suivi de la lyophilisation. MALDI-TOF-MS : *m*/*z* [M+H]⁺ calculé 1245,7 ; trouvé 1245,8 ; [M+Na]⁺ calculé 1267,7 ; trouvé 1267,8.

Caractérisation du composé 11 : MALDI-TOF-MS : *m*/*z* [M+H]⁺ calculé 957,3 ; trouvé 957,8 ; [M+Na]⁺ calculé 979,3 ; trouvé 979,7.

### EXEMPLE 9 : Analyse et quantification de l'expression des molécules de classe II du Complexe Majeur d'Histocompatibilité (CMH), à la surface des cellules, induite par incubation de ces cellules avec des lipopeptides conformes à l'invention.

### 1) Lipopeptides utilisés.

Les lipopeptides conformes à l'invention, qui sont utilisés dans ces expériences, sont les lipopeptides L-Mu-IFNγ a et L-Mu-IFNγ b, dont la synthèse est décrite au paragraphe 3 de l'exemple 5 ci-avant. Comme rappelé au paragraphe 3 de l'exemple 1 ci-avant, le peptide Mu-IFNγ a correspond aux 20 acides aminés contigus de l'extrémité C-terminale de l'interféron-γ (IFN-γ) murin. Le peptide Mu-IFNγ b est une séquence « scramble » de la séquence présente dans le peptide Mu-IFNγ a, c'est-à-dire une séquence dans laquelle les acides aminés (leur ordre dans la séquence) ont été mélangés.

Le lipopeptide L-mIFNγ 95-132 est décrit dans l'article de K. THIAM et al. paru dans J. Med. Chem., 1999, 42, 3732-3736. Il résulte du couplage d'un résidu palmitoyle, porté par une lysine, au niveau de l'extrémité N-tenninale de la séquence 95-132 correspondant aux 38 acides aminés contigus de l'extrémité C-terminale de l'interféron-γ murin. Le couplage de la partie lipophile sur la séquence peptidique est décrite dans l'article de K. THIAM et al. précité : il résulte de l'introduction, en phase solide, du composé Fmoc-Lys(palmitoyle)-OH. Le lipopeptide ainsi obtenu est ensuite déprotégé et séparé du support solide.

Le lipopeptide SL-mIFNγ 95-132 est également décrit dans l'article de K. THIAM et al. précité : il s'agit d'une version « scramble » du lipopeptide L-mIFNγ 95-132.

Le lipopeptide L-mIFNγ 113-132 est également décrit dans l'article de K. THIAM et al. précité : ce lipopeptide comprend la même séquence peptidique que le lipopeptide L-Mu-IFNγ a conforme à l'invention, mais le résidu palmitoyle n'est pas introduit de la même façon que pour le lipopeptide L-Mu-IFNγ a, et le lien entre la séquence peptidique et la partie lipophile n'est pas le même dans les deux lipopeptides (liaison amide entre l'acide palmitique et la fonction amine de la chaîne latérale d'une lysine dans le cas de L-mIFNγ 113-132, le lipopeptide étant synthétisé en phase solide, alors qu'il s'agit d'un lien hydrazone et d'un couplage en solution dans le cas de L-Mu-IFNγ a).

### 2) Protocole de quantification de l'expression des molécules de classe II du Complexe Majeur d'Histocompatibilité (CMH) à la surface des cellules.

Les cellules COLO 205 (lignée cellulaire humaine issue d'un carcinome de colon) proviennent de la banque de cellules ATCC (American Type Culture Collection). Elles sont cultivées en milieu RPMI 1640 (Gibco BRL, Courbevoie, France) avec 10% de sérum de veau foetal (SVF) et 5 mM de pyruvate de sodium, et incubées à 37°C en présence de 5% de CO₂. Les cellules sont stimulées pendant 24 heures avec différentes concentrations (35, 42 ou 50 µM) de lipopeptides. Les cellules sont ensuite marquées pendant 1 heure avec 10 microL d'anticorps de souris anti-HLA DR de classe II dans 10% de SVF/PBS, puis lavées 3 fois par du SVF/PBS, en prenant soit un anticorps couplé au FITC (clone TAL, 1B5, Cymbus Biotechnology Ltd, Hants, Grande-Bretagne) (résultats présentés en Tableau III a), soit l'anticorps L243 révélé par un deuxième anticorps couplé à l'ALEXA 488 (Molecular Probe, Netherland) (résultats présentés en Tableau III b).

L'expression de surface des molécules de classe II du CMH est analysée par cytométrie de flux sur un cytomètre Coulter EPICS II, à raison de 10 000 évènements par échantillon. L'intensité de fluorescence observée est directement proportionnelle à la quantité de molécules de classe II du CMH présente à la surface des cellules. La moyenne de fluorescence observée pour les cellules non traitées et pour les cellules traitées (colonne « Moyenne » dans le tableau III) permet de calculer un rapport entre la moyenne de fluorescence des cellules traitées et la moyenne de fluorescence des cellules non traitées (colonne « Rapport » dans le tableau III). Le contrôle consiste à incuber les cellules en absence de lipopeptides.

### 3) Résultats.

Les résultats obtenus lors de deux séries d'expériences indépendantes sont présentés dans les tableaux III a et III b.

**Tableau III a**

| **Nom du** produit | 35 µM | | 42 µM | | 50 µM | |
|---|---|---|---|---|---|---|
| | Moyenne | Rapport | Moyenne | Rapport | Moyenne | Rapport |
| contrôle | 0,726 | | | | | |
| L-mIFNγ 95-132 | 7,94 | 10,9 | 6,94 | 9,55 | 10,3 | 14,1 |
| SL-mIFNγ 95-132 | 2,23 | 3,07 | 2,30 | 3,16 | 2,69 | 3,70 |
| L-Mu-IFNγ a | 11,1 | 15,2 | 16,7 | 23,0 | 19,5 | 26,8 |
| **L-Mu-IFNγ b** | 3,44 | 4,73 | 5,26 | 7,24 | 7,79 | 10,7 |
| L-mIFNγ 113-132 | 13,2 | 18,18 | 16,1 | 22,1 | 14,6 | 20,11 |

**Tableau III b**

| **Nom du produit** | **5µM** | | **10µM** | | **35µM** | |
|---|---|---|---|---|---|---|
| | **Moyenne** | **Rapport** | **Moyenne** | **Rapport** | **Moyenne** | **Rapport** |
| contrôle | 0.607 | | | | | |
| **L-Mu-IFNγ a** | 0.99 | 1.6 | 1.51 | 2.48 | 5.85 | 9.6 |
| **L-Mu-IFNγ b** | 0.76 | 1.25 | 0.99 | 1.63 | 1.65 | 2.71 |
| **L-Mu-IFNγ c** | 1.3 | 2.14 | 1.72 | 2.83 | 15.1 | 24.8 |
| **L-Mu-IFNγ d** | 1.39 | 2.28 | 2.36 | 3.88 | 7.31 | 12.0 |
| **Cho-Mu-IFNγ a** | 2.04 | 3.4 | 2.81 | 4.6 | 36.9 | 60.7 |
| **Cho-Mu-IFNg b** | 0.95 | 1.56 | 1.88 | 3.1 | 11.6 | 19.1 |
| **Cho-Mu-IFN-γ c** | 2.95 | 4.8 | 4.27 | 7.03 | 21.6 | 35.5 |
| **Cho-Mu-IFNγ d** | 2.02 | 3.32 | 2 | 3.3 | 1.56 | 2.57 |

Dans le tableau III a, la comparaison entre les résultats obtenus pour L-Mu-IFNγ a et pour L-mIFNγ 113-132 (lipopeptides de même taille, comprenant la même séquence peptidique mais différant l'un de l'autre par la nature de la liaison entre la partie lipophile et la séquence peptidique) à 42 µM et à 50 µM montre que l'expression des molécules de classe II du CMH à la surface des cellules est supérieure lorsque les cellules ont été incubées avec le lipopeptide conforme à l'invention.

Dans le tableau III b, la comparaison entre les résultats obtenus avec le produit L-Mu-IFNγ a décrit également en tableau III a et les dérivés comportant une partie lipophile dérivée du cholestérol Cho-Mu-IFNγ a-d montre que l'expression des molécules de classe II à la surface des cellules est encore plus forte lorsque les cellules ont été incubées avec des dérivés du cholestérol qu'avec des dérivés de l'acide palmitique.

Le passage membranaire des lipopeptides conformes à l'invention est donc plus efficace, eu égard à la nature de la liaison entre la partie lipophile et la séquence peptidique (lien hydrazone).

Ces résultats montrent que les lipopeptides conformes à l'invention sont capables de passer à travers la membrane des cellules, de façon plus efficace que les lipopeptides selon l'art antérieur. Les lipopeptides conformes à l'invention sont donc adaptés à la vectorisation de principes actifs dans les cellules.

## Revendications

1. Procédé de couplage, en solution, entre au moins un composé peptidique et au moins un vecteur lipophile portant une fonction aldéhyde, ledit couplage comprenant une étape de réalisation d'un lien hydrazone entre ledit composé peptidique et ledit vecteur lipophile, **caractérisé en ce que** ledit vecteur lipophile est de nature non peptidique et répond à la formule générale (I) :
[(R¹)(R²)ᵢ]D-CHO (I)
dans laquelle :
- i représente 0 ou 1,
- dans le cas où i est égal à 0, D représente une liaison,
- dans le cas où i est égal à 1, D représente un hétérocycle saturé, insaturé ou aromatique, mono- ou polycyclique, et
- R¹ et R², qui peuvent être identiques ou différents, représentent chacun un groupement de formule L-f-E-f' où L représente un résidu d'un lipide, E représente un bras espaceur, et f et f' représentent des fonctions liant, respectivement, L à E et E à D.

2. Procédé selon la revendication 1, **caractérisé en ce qu'**il comprend les étapes suivantes :
a) préparation d'au moins un composé peptidique totalement déprotégé portant, soit au niveau de son extrémité N-terminale, soit au niveau de l'extrémité de la chaîne latérale d'une lysine ou d'une ornithine éventuellement présente à un endroit quelconque de la séquence peptidique, 1 à 4 groupements dérivés d'hydrazine de formule -CO-CHR'-NR-NH₂, où R et R' représentent, indépendamment l'un de l'autre, un atome d'hydrogène ou un groupement alkyle saturé ou insaturé, linéaire, ramifié ou cyclique, comprenant de 1 à 10 atomes de carbone, ainsi qu'éventuellement 1 à 3 hétéroatomes choisis parmi l'oxygène, le soufre et l'azote, et pouvant être substitué par 1 à 6 groupements choisis parmi les groupes hydroxy, alkoxy, aryloxy, amino, aminoalkyle, aminoaryle, thio, thioalkyle, carbonyle, carboxyle, guanidino et carboxamido ;
b) réaction, en solution, du(des)dit(s) composé(s) peptidique(s) obtenu(s) à l'étape a) avec au moins un vecteur lipophile de nature non peptidique de formule (I) telle que définie dans la revendication 1, porteur d'une fonction aldéhyde.

3. Procédé selon la revendication 2, **caractérisé en ce que** la réaction effectuée à l'étape b) est réalisée dans un mélange d'eau et d'au moins un solvant lipophile miscible à l'eau.

4. Procédé selon la revendication 3, **caractérisé en ce que** ledit solvant lipophile miscible à l'eau est le tert-butanol.

5. Procédé selon l'une quelconque des revendications 2 à 4, **caractérisé en ce que** lesdits groupements dérivés d'hydrazine sont des groupements α-hydrazinoacétiques.

6. Procédé selon l'une quelconque des revendications précédentes, **caractérisé en ce que** ledit composé peptidique est sélectionné dans le groupe constitué par les peptides et les dérivés de peptides.

7. Procédé selon la revendication 6, **caractérisé en ce que** lesdits dérivés de peptides sont choisis parmi les glycopeptides, les pseudopeptides et les glycomimétiques dendrimériques de nature peptidique.

8. Procédé selon la revendication 7, **caractérisé en ce que** lesdits glycomimétiques dendrimériques de nature peptidique répondent à la formule générale (II) ci-après :
(B²M)ₘ(XN)ₙA (II)
dans laquelle :
- B² répond à une ou plusieurs formules générales (a) ou (b) ci-après : 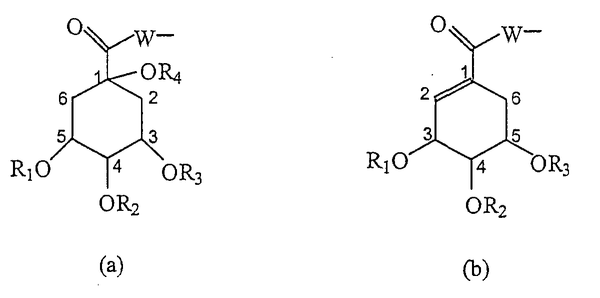
où R₁, R₂, R₃ et R₄ représentent, indépendamment les uns des autres, un atome d'hydrogène ou un groupe protecteur, et où W représente une liaison ou une chaîne carbonée linéaire, ramifiée ou cyclique, saturée ou insaturée, comprenant de 1 à 18 atomes de carbone et éventuellement de 1 à 12 atomes choisis parmi l'oxygène, le soufre et l'azote, ladite chaîne carbonée étant éventuellement substituée par 1 à 16 atomes d'halogène,
- X représente le reste d'un oligopeptide X' comprenant de 1 à 6 acides aminés,
- A représente le reste d'un composé A' au moins trifonctionnel,
- m est un nombre entier compris entre 1 et 32,
- n est un nombre entier compris entre 0 et 32, et
- M et N représentent chacun un groupe de liaison, respectivement entre B² et A quand n = 0 ou B² et X quand n est différent de 0, et entre X et A quand n est différent de 0, et comprennent, indépendamment l'un de l'autre, une fonction choisie parmi les fonctions oxime, hydrazone, amide, ester, thioester, hydrazide, hydroxamate, éther, thioéther, amine, carbonate, carbamate, thiocarbonate, thiocarbamate, urée, thiourée et thiazolidine.

9. Procédé selon la revendication 8, **caractérisé en ce que** le composé A' comprend un enchaînement d'acides aminés, identiques ou différents, sélectionnés dans le groupe constitué par la lysine, l'hydroxylysine, la sérine, la thréonine, la cystéine, l'omithine, l'acide aspartique et l'acide glutamique.

10. Procédé selon la revendication 8 ou la revendication 9, **caractérisé en ce que** le composé de formule générale (II) est tel que m est un nombre entier compris entre 4 et 16, n est un nombre entier compris entre 2 et 8, X représente le reste d'un oligopeptide comprenant de 2 à 4 résidus d'acides aminés, tandis que A représente le reste d'un enchaînement comprenant de 2 à 8 résidus de lysine et se présentant sous la forme d'un dendrimère.

11. Procédé selon l'une quelconque des revendications 8 à 10, **caractérisé en ce que** le composé de formule générale (II) est tel que B² représente un reste d'un ou plusieurs composés choisis parmi l'acide (-)-shikimique et l'acide (-)-quinique.

12. Procédé selon l'une quelconque des revendications précédentes, **caractérisé en ce qu'**il met en oeuvre plusieurs composés peptidiques différents et/ou plusieurs vecteurs lipophiles différents.

13. Procédé selon l'une quelconque des revendications 2 à 12, **caractérisé en ce qu'**il met en oeuvre lors de l'étape b), au moins un vecteur lipophile portant une fonction aldéhyde, au moins un composé peptidique choisi parmi les peptides, les glycopeptides et les pseudopeptides, ainsi qu'au moins un glycomimétique dendrimérique de formule générale (II) telle que définie dans l'une quelconque des revendications 8 à 11.

14. Vecteur lipophile utilisable dans le procédé selon l'une quelconque des revendications précédentes, **caractérisé en ce qu'**il répond à la formule générale (I) :
[(R¹)(R²)ᵢ]D-CHO (I)
telle que définie dans la revendication 1.

15. Vecteur lipophile selon la revendication 14, **caractérisé en ce que** i est égal à 1 et **en ce que** D représente l'hétérocycle 1-aza-3,7-dioxabicyclo (3.3.0)-octane.

16. Vecteur lipophile selon la revendication 14 ou la revendication 15, **caractérisé en ce que** L représente un stérol, un dérivé de stérol ou une chaîne carbonée saturée ou insaturée, linéaire ou ramifiée, comprenant entre 4 et 30 atomes de carbone.

17. Vecteur lipophile selon la revendication 16, **caractérisé en ce que** ledit dérivé de stérol est un dérivé du cholestérol et **en ce que** ladite chaîne carbonée répond à la formule CH₃-(CH₂)₁₄- ou CH₃-(CH₂)₇-CH=CH-(CH₂)₇-.

18. Vecteur lipophile selon l'une quelconque des revendications 14 à 17, **caractérisé en ce que** E représente une chaîne carbonée linéaire, ramifiée ou cyclique, saturée ou insaturée, comportant de 1 à 18 atomes de carbone et, éventuellement, 1 à 16 hétéroatomes et/ou 1 à 7 groupements sélectionnés parmi les groupements carbonyles, les hétérocycles, les hétéroaryles, les carbocycles et les aryles.

19. Vecteur lipophile selon la revendication 18, **caractérisé en ce que** E est substitué par 1 à 8 groupements hydroxyles ou amino et/ou 1 à 16 atomes d'halogène.

20. Vecteur lipophile selon l'une quelconque des revendications 14 à 19, **caractérisé en ce que** f représente une liaison -CO-NH- ou -CO-O- et f' représente une liaison -NH-CO- ou -O-CO-.

21. °) Vecteur lipophile selon l'une quelconque des revendications 14 à 20, **caractérisé en ce qu'**il répond à la formule (III) ou à la formule (IV) : 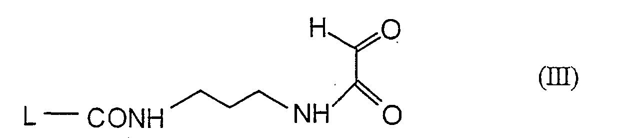 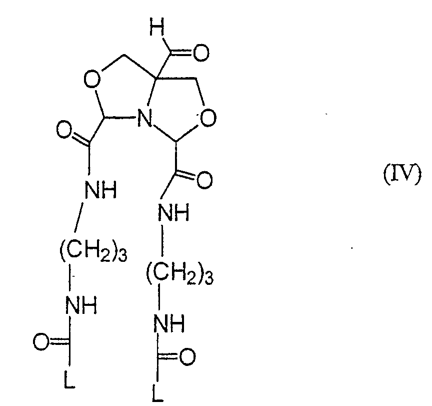 dans lesquelles L est tel que défini dans l'une quelconque des revendication 1, 16 ou 17.

22. Vecteur lipophile selon la revendication 21, **caractérisé en ce que** L représente une chaîne carbonée de formule CH₃-(CH₂)₁₄- dans les formules (III) et (IV).

23. Vecteur lipophile selon la revendication 21, **caractérisé en ce que** L représente une chaîne carbonée de formule CH₃-(CH₂)₇-CH=CH-(CH₂)₇- dans la formule (III).

24. Lipopeptide susceptible d'être obtenu par le procédé de couplage tel que défini dans l'une quelconque des revendications 1 à 13, **caractérisé en ce qu'**il est essentiellement constitué d'au moins un composé peptidique lié, par un lien hydrazone, à au moins un vecteur lipophile de nature non peptidique de formule (I) tel que défini dans l'une quelconque des revendications 14 à 23.

25. Mélange de plusieurs lipopeptides différents tels que définis dans la revendication 24.

26. Utilisation d'un lipopeptide selon la revendication 24 pour le ciblage cellulaire.

27. Utilisation du procédé selon l'une quelconque des revendications 1 à 13 pour la préparation d'un médicament comprenant au moins un principe actif de nature peptidique vectorisé par au moins un composé lipophile, utile pour le ciblage cellulaire.

## Patentansprüche

1. Verfahren zur Kupplung in Lösung zwischen wenigstens einer Peptidverbindung und wenigstens einem lipophilen Vektor, der eine Aldehydfunktion aufweist, wobei die Kupplung einen Schritt der Ausbildung einer Hydrazonbindung bzw. eines Hydrazonbindegliedes zwischen der Peptidverbindung und dem lipophilen Vektor umfasst, **dadurch gekennzeichnet, dass** der lipophile Vektor von nicht-peptidartiger Beschaffenheit ist und durch die allgemeine Formel (I) wiedergegeben wird:
[(R¹)(R²)ᵢ]D-CHO (I)
in welcher:
- i 0 oder 1 bedeutet,
- wenn i 0 ist, D eine Bindung bedeutet,
- wenn i 1 ist, D einen gesättigten, ungesättigten oder aromatischen, monooder polycyclischen Heterocyclus bedeutet, und
- R¹ und R², welche gleich oder verschieden sein können, jeweils eine Gruppe der Formel L-f-E-f bedeuten, wobei L einen Lipidrest bedeutet, E einen Spacerarm bzw. Abstandshalter bedeutet, und f und f Funktionen bedeuten, die L mit E bzw. E mit D verbinden.

2. Verfahren nach Anspruch 1, **dadurch gekennzeichnet, dass** es die folgenden Schritte umfasst:
a) Herstellung wenigstens einer Peptidverbindung, von der alle Schutzgruppen entfernt sind und die entweder an ihrem N-terminalen Ende oder am Ende der Seitenkette eines gegebenenfalls an einem beliebigen Ort der Peptidsequenz vorhandenen Lysins oder Ornithins 1 bis 4 von Hydrazin abgeleitete Gruppen der Formel -CO-CHR'-NR-NH₂ aufweist, wobei R und R' unabhängig voneinander ein Wasserstoffatom oder eine gesättigte oder ungesättigte, lineare, verzweigte oder cyclische Alkylgruppe bedeuten, die 1 bis 10 Kohlenstoffatome sowie gegebenenfalls 1 bis 3 Heteroatome, ausgewählt aus Sauerstoff, Schwefel und Stickstoff, umfasst, und durch 1 bis 6 Gruppen, ausgewählt aus Hydroxy-, Alkoxy-, Aryloxy-, Amino-, Aminoalkyl-, Aminoaryl-, Thio-, Thioalkyl-, Carbonyl-, Carboxyl-, Guanidino- und Carboxamidogruppen, substituiert sein kann;
b) Reaktion in Lösung der im Schritt a) erhaltenen Peptidverbindung(en) mit wenigstens einem lipophilen Vektor von nicht-peptidartiger Beschaffenheit der Formel (I), wie sie in Anspruch 1 definiert ist, welcher eine Aldehydfunktion aufweist.

3. Verfahren nach Anspruch 2, **dadurch gekennzeichnet, dass** die im Schritt b) bewirkte Reaktion in einem Gemisch aus Wasser und wenigstens einem mit Wasser mischbaren lipophilen Lösungsmittel durchgeführt wird.

4. Verfahren nach Anspruch 3, **dadurch gekennzeichnet, dass** das mit Wasser mischbare lipophile Lösungsmittel tert-Butanol ist.

5. Verfahren nach einem der Ansprüche 2 bis 4, **dadurch gekennzeichnet, dass** die von Hydrazin abgeleiteten Gruppen α-Hydrazinoessigsäuregruppen sind.

6. Verfahren nach einem der vorangehenden Ansprüche, **dadurch gekennzeichnet, dass** die Peptidverbindung ausgewählt wird aus der Gruppe bestehend aus den Peptiden und den Peptidderivaten.

7. Verfahren nach Anspruch 6, **dadurch gekennzeichnet, dass** die Peptidderivate ausgewählt sind aus den Glycopeptiden, den Pseudopeptiden und den dendrimeren Glykomimetika mit peptidartiger Beschaffenheit.

8. Verfahren nach Anspruch 7, **dadurch gekennzeichnet, dass** die dendrimeren Glykomimetika mit peptidartiger Beschaffenheit durch die nachstehende allgemeine Formel (II) wiedergegeben werden:
(B²M)ₘ(XN)ₙA (II)
in welcher:
- B² durch eine oder mehrere nachstehende allgemeine Formeln (a) oder (b) wiedergegeben wird: 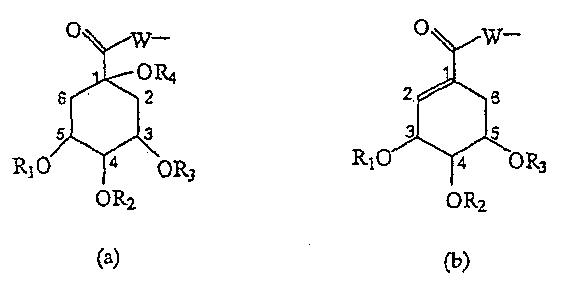
wobei R₁, R₂, R₃ und R₄ unabhängig voneinander ein Wasserstoffatom oder eine Schutzgruppe bedeuten und wobei W eine Bindung oder eine lineare, verzweigte oder cyclische, gesättigte oder ungesättigte Kohlenstoffkette umfasst, die 1 bis 18 Kohlenstoffatome und gegebenenfalls 1 bis 12 Atome, ausgewählt aus Sauerstoff, Schwefel und Stickstoff, umfasst, wobei die Kohlenstoffkette gegebenenfalls durch 1 bis 16 Halogenatome substituiert ist,
- X den Rest eines Oligopeptids X' bedeutet, umfassend 1 bis 6 Aminosäuren,
- A den Rest einer Verbindung A' bedeutet, welche mindestens trifunktionell ist,
- m eine ganze Zahl zwischen 1 und 32 ist,
- n eine ganze Zahl zwischen 0 und 32 ist, und
- M und N jeweils eine Bindungsgruppe zwischen B² und A, wenn n = 0, oder B² und X, wenn n von 0 verschieden ist, bzw. zwischen X und A, wenn n von 0 verschieden ist, bedeuten und unabhängig voneinander eine Funktion umfassen, die ausgewählt wird aus den Funktionen Oxim, Hydrazon, Amid, Ester, Thioester, Hydrazid, Hydroxamat, Ether, Thioether, Amin, Carbonat, Carbamat, Thiocarbonat, Thiocarbamat, Harnstoff, Thioharnstoff und Thiazolidin.

9. Verfahren nach Anspruch 8, **dadurch gekennzeichnet, dass** die Verbindung A' eine Verkettung von gleichen oder verschiedenen Aminosäuren, ausgewählt aus der Gruppe bestehend aus Lysin, Hydroxylysin, Serin, Threonin, Cystein, Ornithin, Asparaginsäure und Glutaminsäure, umfasst.

10. Verfahren nach Anspruch 8 oder Anspruch 9, **dadurch gekennzeichnet, dass** die Verbindung der allgemeinen Formel (II) so ist, dass m eine ganze Zahl zwischen 4 und 16 ist, n eine ganze Zahl zwischen 2 und 8 ist, X den Rest eines Oligopeptids bedeutet, das 2 bis 4 Aminosäurereste umfasst, während A den Rest einer Verkettung bedeutet, die 2 bis 8 Lysinreste umfasst und in Form eines Dendrimers vorliegt.

11. Verfahren nach einem der Ansprüche 8 bis 10, **dadurch gekennzeichnet, dass** die Verbindung der allgemeinen Formel (II) so ist, dass B² einen Rest von einer oder mehreren Verbindungen, ausgewählt aus (-)-Shikimisäure und (-)-Chinasäure, bedeutet.

12. Verfahren nach einem der vorangehenden Ansprüche, **dadurch gekennzeichnet, dass** es mehrere verschiedene Peptidverbindungen und/oder mehrere verschiedene lipophile Vektoren einsetzt.

13. Verfahren nach einem der Ansprüche 2 bis 12, **dadurch gekennzeichnet, dass** es in dem Schritt b) wenigstens einen lipophilen Vektor, der eine Aldehydfunktion aufweist, wenigstens eine Peptidverbindung, ausgewählt aus den Peptiden, den Glycopeptiden und den Pseudopeptiden, sowie wenigstens ein dendrimeres Glykomimetikum der allgemeinen Formel (11), wie sie in einem der Ansprüche 8 bis 11 definiert ist, einsetzt.

14. Lipophiler Vektor, der in dem Verfahren nach einem der vorangehenden Ansprüche verwendet werden kann, **dadurch gekennzeichnet, dass** er durch die allgemeine Formel (I) wiedergegeben wird:
[(R¹)(R²)ᵢ]D-CHO (I)
wie sie in Anspruch 1 definiert ist.

15. Lipophiler Vektor nach Anspruch 14, **dadurch gekennzeichnet, dass** i 1 ist und dass D den Heterocyclus 1-Aza-3,7-dioxabicyclo[3.3.0]-octan bedeutet.

16. Lipophiler Vektor nach Anspruch 14 oder Anspruch 15, **dadurch gekennzeichnet, dass** L ein Sterol, ein Sterolderivat oder eine gesättigte oder ungesättigte, lineare oder verzweigte Kohlenstoffkette, die zwischen 4 und 30 Kohlenstoffatome umfasst, bedeutet.

17. Lipophiler Vektor nach Anspruch 16, **dadurch gekennzeichnet, dass** das Sterolderivat ein Derivat von Cholesterol ist und dass die Kohlenstoffkette durch die Formel CH₃-(CH₂)₁₄- oder CH₃-(CH₂)₇-CH=CH-(CH₂)₇- wiedergegeben wird.

18. Lipophiler Vektor nach einem der Ansprüche 14 bis 17, **dadurch gekennzeichnet, dass** E eine lineare, verzweigte oder cyclische, gesättigte oder ungesättigte Kohlenstoffkette bedeutet, die 1 bis 18 Kohlenstoffatome und gegebenenfalls 1 bis 16 Heteroatome und/oder 1 bis 7 Gruppen, ausgewählt aus den Carbonylgruppen, den Heterocyclen, den Heteroarylen, den Carbocyclen und den Arylen, umfasst.

19. Lipophiler Vektor nach Anspruch 18, **dadurch gekennzeichnet, dass** E durch 1 bis 8 Hydroxyl- oder Aminogruppen und/oder 1 bis 16 Halogenatome substituiert ist.

20. Lipophiler Vektor nach einem der Ansprüche 14 bis 19, **dadurch gekennzeichnet, dass** f eine -CO-NH- oder -CO-O- -Bindung bedeutet und f' eine -NH-CO- oder -O-CO- -Bindung bedeutet.

21. Lipophiler Vektor nach einem der Ansprüche 14 bis 20, **dadurch gekennzeichnet, dass** er durch die Formel (III) oder die Formel (IV) wiedergegeben wird: 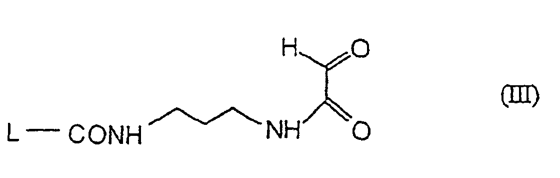 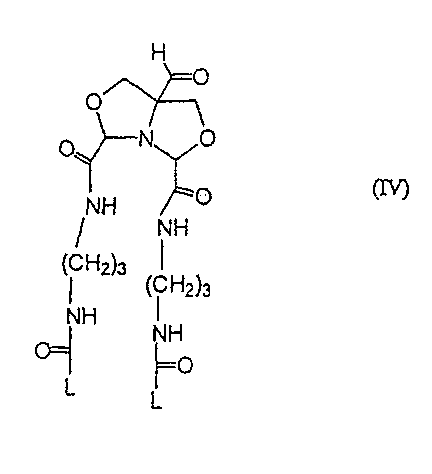 in welchen L wie in einem der Ansprüche 1, 16 oder 17 definiert ist.

22. Lipophiler Vektor nach Anspruch 21, **dadurch gekennzeichnet, dass** L eine Kohlenstoffkette der Formel CH₃-(CH₂)₁₄- in den Formeln (III) und (IV) bedeutet.

23. Lipophiler Vektor nach Anspruch 21, **dadurch gekennzeichnet, dass** L eine Kohlenstoffkette der Formel CH₃-(CH₂)₇-CH=CH-(CH₂)₇- in der Formel (III) bedeutet.

24. Lipopeptid, das durch das Verfahren zur Kupplung, wie es in einem der Ansprüche 1 bis 13 definiert ist, erhalten werden kann, **dadurch gekennzeichnet, dass** es im Wesentlichen aus wenigstens einer Peptidverbindung besteht, die über eine Hydrazonbindung bzw. ein Hydrazonbindeglied an wenigstens einen lipophilen Vektor mit nicht-peptidartiger Beschaffenheit der Formel (I), wie er in einem der Ansprüche 14 bis 23 definiert ist, gebunden ist.

25. Gemisch aus mehreren verschiedenen Lipopeptiden, wie sie in Anspruch 24 definiert sind.

26. Verwendung eines Lipopeptids nach Anspruch 24 zur gezielten Ansteuerung von Zellen.

27. Verwendung des Verfahrens nach einem der Ansprüche 1 bis 13 zur Herstellung eines Medikaments, welches wenigstens einen Wirkstoff mit peptidartiger Beschaffenheit, der durch wenigstens eine lipophile Verbindung vektorisiert ist, umfasst, das zur gezielten Ansteuerung von Zellen brauchbar ist.

## Claims

1. A process for coupling, in solution, between at least one peptide compound and at least one lipophilic vector bearing an aldehyde function, said coupling comprising a step for producing a hydrazone bond between said peptide compound and said lipophilic vector, **characterized in that** said lipophilic vector is nonpeptide in nature and corresponds to general formula (I):
[(R¹)(R²)₁]D-CHO (I)
in which:
- i represents 0 or 1,
- when i is equal to 0, D represents a bond,
- when i is equal to 1, D represents a saturated, unsaturated or aromatic, monocyclic or polycyclic heterocycle, and
- R¹ and R², which may be identical or different, each represent a group of formula L-f-E-f' where L represents a residue of a lipid, E represents a spacer arm, and f and f' represent functions bonding, respectively, L to E and E to D.

2. A process according to claim 1, **characterized in that** it comprises the following steps:
a) preparation of at least one completely deprotected peptide compound bearing, either at its N-terminal end or at the end of the side chain of a lysine or of an ornithine optionally present at any site on the peptide sequence, 1 to 4 hydrazine-derived groups of formula -CO-CHR'-NR-NH₂, where R and R' represent, independently of one another, a hydrogen atom or a saturated or unsaturated, linear, branched or cyclic alkyl group comprising from 1 to 10 carbon atoms, and also, optionally, 1 to 3 hetero atoms chosen from oxygen, sulphur and nitrogen, and which may be substituted with 1 to 6 groups chosen from hydroxyl, alkoxy, aryloxy, amino, aminoalkyl, aminoaryl, thio, thioalkyl, carbonyl, carboxyl, guanidino and carboxamido groups;
b) reaction, in solution, of said peptide compound(s) obtained in step a) with at least one lipophilic vector which is nonpeptide in nature, of formula (I) as defined in claim 1, bearing an aldehyde function.

3. A process according to claim 2, **characterized in that** the reaction performed in step b) is carried out in a mixture of water and at least one water-miscible lipophilic solvent.

4. A process according to claim 3, **characterized in that** said water-miscible lipophilic solvent is tert-butanol.

5. A process according to any one of claims 2 to 4, **characterized in that** said hydrazine-derived groups are α-hydrazinoacetic groups.

6. A process according to any one of the preceding claims, **characterized in that** said peptide compound is selected from the group consisting of peptides and peptide derivatives.

7. A process according to claim 6, **characterized in that** said peptide derivatives are chosen from glycopeptides, pseudopeptides and dendrimeric glycomimetics which are peptide in nature.

8. A process according to claim 7, **characterized in that** said dendrimeric glycomimetics which are peptide in nature correspond to general formula (II) below:
(B²M)ₘ (XN)ₙA (II)
in which:
- B² corresponds to one or more general formulae (a) or (b) below: 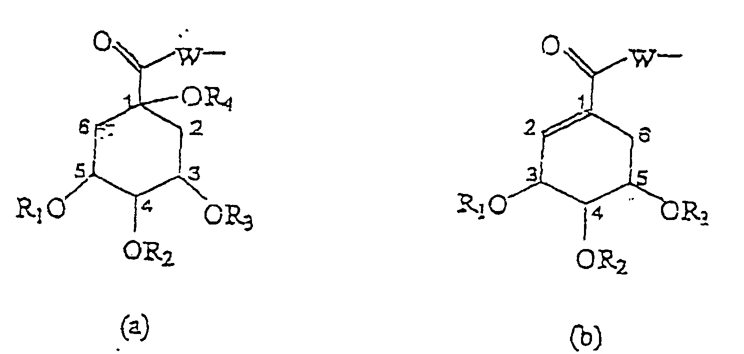
where R₁, R₂, R₃ and R₄ represent, independently of one another, a hydrogen atom or a protective group, and where W represents a bond or a saturated or unsaturated, linear, branched or cyclic carbon chain comprising from 1 to 18 carbon atoms and, optionally, from 1 to 12 atoms chosen from oxygen, sulphur and nitrogen, said carbon chain being optionally substituted with 1 to 16 halogen atoms,
- X represents the residue of an oligopeptide X' comprising from 1 to 6 amino acids,
- A represents the residue of a compound A' which is at least trifunctional,
- m is an integer from 1 to 32,
- n is an integer from 0 to 32, and
- M and N each represent a bonding group, respectively between B² and A when n = 0, or B² and X when n is other than 0, and between X and A when n is other than 0, and comprise, independently of one another, a function chosen from oxime, hydrazone, amide, ester, thioester, hydrazide, hydroxamate, ether, thioether, amine, carbonate, carbamate, thiocarbonate, thiocarbamate, urea, thiourea and thiazolidine functions.

9. A process according to claim 8, **characterized in that** the compound A' comprises a series of amino acids, which may be identical or different, selected from the group consisting of lysine, hydroxylysine, serine, threonine, cysteine, ornithine, aspartic acid and glutamic acid.

10. A process according to claim 8 or claim 9, **characterized in that** the compound of general formula (II) is such that m is an integer from 4 to 16, n is an integer from 2 to 8, X represents the residue of an oligopeptide comprising from 2 to 4 amino acid residues, while A represents the residue of a series comprising from 2 to 8 lysine residues and is in the form of a dendrimer.

11. A process according to any one of claims 8 to 10, **characterized in that** the compound of general formula (II) is such that B² represents a residue of one or more compounds chosen from (-)-shikimic acid and (-)-quinic acid.

12. A process according to any one of the preceding claims, **characterized in that** it uses several different peptide compounds and/or several different lipophilic vectors.

13. A process according to any one of claims 2 to 12, **characterized in that** it uses, in step b), at least one lipophilic vector bearing an aldehyde function, at least one peptide compound chosen from peptides, glycopeptides and pseudopeptides, and also at least one dendrimeric glycomimetic of the general formula (II) as defined in any one of claims 8 to 11.

14. A lipophilic vector which can be used in the process according to any one of the preceding claims, **characterized in that** it corresponds to the general formula (I):
[(R¹)(R²)ᵢ]D-CHO (I)
as defined in claim 1.

15. A lipophilic vector according to claim 14, **characterized in that** i is equal to 1 and **in that** D represents the heterocycle 1-aza-3,7-dioxabicyclo(3.3.0)octane.

16. A lipophilic vector according to claim 14 or claim 15, **characterized in that** L represents a sterol, a sterol derivative or a saturated or unsaturated, linear or branched carbon chain comprising from 4 to 30 carbon atoms.

17. A lipophilic vector according to claim 16, **characterized in that** said sterol derivative is a cholesterol derivative and **in that** said carbon chain corresponds to formula CH₃-(CH₂)₁₄- or CH₃-(CH₂)₇-CH=CH-(CH₂)₇-.

18. A lipophilic vector according to any one of claims 14 to 17, **characterized in that** E represents a saturated or unsaturated, linear, branched or cyclic carbon chain comprising from 1 to 18 carbon atoms and, optionally, 1 to 16 hetero atoms and/or 1 to 7 groups selected from carbonyl groups, heterocycles, heteroaryls, carbocycles and aryls.

19. A lipophilic vector according to claim 18, **characterized in that** E is substituted with 1 to 8 hydroxyl or amino groups and/or 1 to 16 halogen atoms.

20. A lipophilic vector according to any one of claims 14 to 19, **characterized in that** f represents a bond -CO-NH- or -CO-O- and f' represents a bond -NH-CO- or -O-CO-.

21. A lipophilic vector according to any one of claims 14 to 20, **characterized in that** it corresponds to formula (III) or to formula (IV): 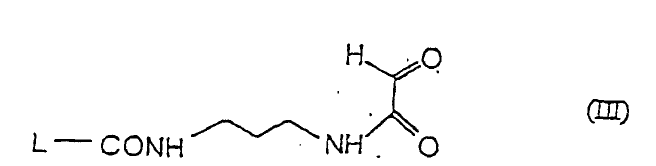 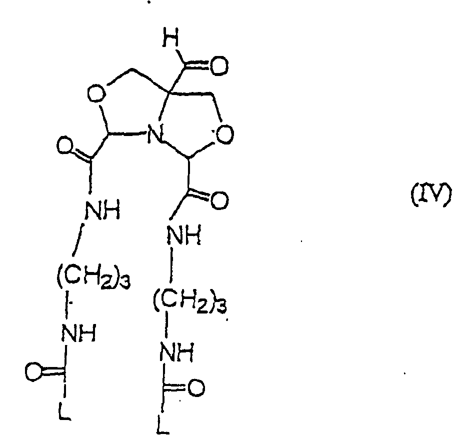 in which L is as defined in any one of claims 1, 16 or 17.

22. A lipophilic vector according to claim 21, **characterized in that** L represents a carbon chain of formula CH₃-(CH₂)₁₄- in formulae (III) and (IV).

23. A lipophilic vector according to claim 21, **characterized in that** L represents a carbon chain of formula CH₃-(CH₂)₇-CH=CH-(CH₂)₇- in formula (III).

24. A lipopeptide which can be obtained by the coupling process as defined in any one of claims 1 to 13, **characterized in that** it essentially consists of at least one peptide compound bonded, via a hydrazone bond, to at least one lipophilic vector which is nonpeptide in nature, of formula (I) as defined in any one of claims 14 to 23.

25. A mixture of several different lipopeptides as defined in claim 24.

26. The use of a lipopeptide according to claim 24, for cell targeting.

27. The use of the process according to any one of claims 1 to 13, for preparing a medicinal product comprising at least one active principle which is peptide in nature, vectorized by at least one lipophilic compound, which is useful for cell targeting.
